(19) Europäisches Patentamt
European Patent Office
Office européen des brevets

(11) **EP 3 140 267 B1**

(12) **EUROPÄISCHE PATENTSCHRIFT**

(45) Veröffentlichungstag und Bekanntmachung des Hinweises auf die Patenterteilung:
**31.03.2021 Patentblatt 2021/13**

(21) Anmeldenummer: **15723452.7**

(22) Anmeldetag: **06.05.2015**

(51) Int Cl.:
*C07C 5/333* (2006.01)  *C07C 15/46* (2006.01)
*B01J 23/00* (2006.01)  *B01J 23/83* (2006.01)
*B01J 23/889* (2006.01)  *B01J 37/08* (2006.01)
*B01J 35/00* (2006.01)  *B01J 35/02* (2006.01)
*B01J 37/04* (2006.01)  *B01J 37/00* (2006.01)
*B01J 35/10* (2006.01)

(86) Internationale Anmeldenummer:
**PCT/EP2015/059907**

(87) Internationale Veröffentlichungsnummer:
**WO 2015/169825 (12.11.2015 Gazette 2015/45)**

(54) **KATALYSATOR FÜR DIE DEHYDRIERUNG VON KOHLENWASSERSTOFFEN**

CATALYST FOR THE DEHYDROGENATION OF HYDROCARBONS

CATALYSEUR POUR LA DÉSHYDROGÉNATION D'HYDROCARBURES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.05.2014 EP 14167665**

(43) Veröffentlichungstag der Anmeldung:
**15.03.2017 Patentblatt 2017/11**

(73) Patentinhaber: **BASF SE**
**67056 Ludwigshafen am Rhein (DE)**

(72) Erfinder:
• **PATCAS, Florina Corina**
**67065 Ludwigshafen (DE)**
• **HINRICHSEN, Bernd**
**70569 Stuttgart (DE)**
• **DIETERLE, Martin**
**67059 Ludwigshafen (DE)**

(56) Entgegenhaltungen:
EP-A1- 1 027 928    WO-A1-97/10898
US-A1- 2013 165 723    US-B1- 6 551 958

• **DULAMITA N ET AL: "Ethylbenzene dehydrogenation on Fe2O3-Cr2O3-K2CO3 catalysts promoted with transitional metal oxides", APPLIED CATALYSIS A: GENERAL, ELSEVIER SCIENCE, AMSTERDAM, NL, Bd. 287, Nr. 1, 15. Juni 2005 (2005-06-15) , Seiten 9-18, XP027814530, ISSN: 0926-860X**
• **LIAO S J ET AL: "Effect of TiO2 on the structure and catalytic behavior of iron-potassium oxide catalyst for dehydrogenation of ethylbenzene to styrene", CATALYSIS COMMUNICATIONS, ELSEVIER SCIENCE, AMSTERDAM, NL, Bd. 9, Nr. 9, 15. Mai 2008 (2008-05-15), Seiten 1817-1821, XP022625412, ISSN: 1566-7367, DOI: 10.1016/J.CATCOM.2008.02.009**

**Beschreibung**

Die vorliegende Erfindung betrifft einen Katalysator zur Dehydrierung von Kohlenwasserstoffen auf Basis von Eisenoxid sowie ein Verfahren zu dessen Herstellung. Der Katalysator enthält mindestens eine Eisenverbindung, mindestens eine Kaliumverbindung und mindestens eine Cerverbindung, wobei die mindestens eine Eisenverbindung und die mindestens eine Kaliumverbindung zumindest teilweise in Form von einer oder mehreren K/Fe-Mischoxidphasen der allgemeinen Formel $K_xFe_yO_z$ vorliegen, wobei x eine Zahl von 1 bis 17 ist; y eine Zahl von 1 bis 22 ist, und z eine Zahl von 2 bis 34 ist, wobei der Katalysator mindestens 20 Gew.-%, bezogen auf den gesamten Katalysator, der K/Fe-Mischoxidphasen enthält und wobei der Katalysator kristallines Cerdioxid enthält, das eine Kristallitgröße im Bereich von 10 nm bis 30 nm aufweist.

Weiterhin betrifft die vorliegende Erfindung ein Verfahren zur katalytischen Dehydrierung von Kohlenwasserstoffen unter Verwendung des erfindungsgemäßen Katalysators.

Im Stand der Technik ist seit langem der Einsatz von Eisenoxid-basierten Katalysatoren bei der Dehydrierung verschiedener Kohlenwasserstoffe zu den entsprechenden ungesättigten Kohlenwasserstoffen bekannt. Großtechnisch sind beispielsweise die Dehydrierungen von Ethylbenzol zu Styrol, von Isopropylbenzol zu alpha-Methylstyrol, von Buten zu Butadien oder von Isoamylen zu Isopren von Bedeutung. Die Herstellung von Styrol durch heterogen katalysierte Dehydrierung von Ethylbenzol in Gegenwart von Wasserdampf ist ein großtechnisch bereits seit Beginn der dreißiger Jahre durchgeführtes Verfahren und hat sich als Syntheseweg für Styrol durchgesetzt. Styrol stellt eines der wichtigsten Monomere der Kunststoffindustrie dar und wird beispielsweise für die Herstellung von Polystyrol, Acrylnitril-Butadien-Styrol-Polymer (ABS) und synthetischem Kautschuk verwendet.

Die im Stand der Technik beschriebenen Eisenoxid-basierten Dehydrier-Katalysatoren sind in der Regel Multikomponenten-Systeme und enthalten im wesentlichen Eisenoxid und eine Alkalimetallverbindung, welche bei der Katalysatorherstellung beispielsweise als Alkalimetalloxid, - carbonat oder -hydroxid eingesetzt wird. Darüber hinaus enthalten diese Katalysatoren in der Regel verschiedene weitere aktive Komponenten (Promotoren), beispielsweise Oxide der Elemente der 5. und 6. Nebengruppe des Periodensystems oder der Seltenen Erden. Beispielsweise ist der Einsatz von Cerverbindungen als Promotor für solche Eisenoxid-basierten Dehydrier-Katalysatoren im Stand der Technik beschrieben.

Die katalytische Dehydrierung von aliphatischen oder alkylaromatischen Kohlenwasserstoffen wird großtechnisch üblicherweise in Gegenwart von Wasserdampf bei Temperaturen im Bereich von 500 bis 700 °C durchgeführt. Typischerweise werden in diesen Verfahren der Kohlenwasserstoff und der Wasserdampf gemischt und bei höheren Temperaturen über den Eisenoxid-Dehydrier-Katalysator geleitet.

Als eine Folge von Koksbildung werden im Laufe des Dehydrier-Verfahrens typischerweise die aktiven Zentren des Dehydrier-Katalysators blockiert, und es kommt zu einer allmählichen Deaktivierung des Katalysators. Um diese Deaktivierung zu vermindern wird dem Kohlenwasserstoff in der Regel Wasserdampf zugesetzt. Durch den Wasserdampf kann der auf der Katalysatoroberfläche entstandenen Koks in-situ vergast werden, wodurch die aktive Katalysatoroberfläche regeneriert werden kann. Daneben hat der Wasserdampf typischerweise noch die folgenden Funktionen: Lieferung der benötigten Reaktionswärme für die endotherme Dehydrierungsreaktion, Verschiebung des Gleichgewichtes zur Produktseite durch Verringerung der partiellen Drücke der Edukte, Aufrechterhaltung des Oxidationszustandes des Eisens gegen die reduzierende Wirkung von Wasserstoff und Kohlenwasserstoff.

Im Stand der Technik sind zahlreiche Dehydrier-Katalysatoren auf der Basis von Eisenoxid beschrieben.

Die wissenschaftliche Publikation Hirano et al. (Appl. Catal. 28, 1986, p. 119-130) untersucht den Einsatz von Cer als Promotor in einem Eisenoxid-Kaliumoxid-Katalysator. Die Publikation beschreibt, dass durch den Zusatz von 5 Gew.-% Cerdioxid in einem Eisenoxid-Kaliumoxid-Katalysator die Aktivierungsenergie bei der Umsetzung von Ethylbenzol zu Styrol abnimmt und die Reaktionsgeschwindigkeit zunimmt.

Das Dokument US 4,460,706 beschreibt Dehydrier-Katalysatoren enthaltend 40 bis 87,5 Gew.-% $Fe_2O_3$, 11 bis 50 Gew.-% Ceroxid (gerechnet als $Ce_2O_3$) und 1,5 bis 40 Gew.-% $K_2O$. US 4,460,706 beschreibt experimentelle Beispiele, bei denen Katalysatoren mit einem Gehalt von 16,7 Gew.-% $Ce_2O_3$ unter verschiedenen Kalzinierungsbedingungen hergestellt werden. Es wird eine Kalzinierung-Dauer von 3 h und ein bevorzugter Temperaturbereich für das Kalzinieren von 900 bis 1100°C beschrieben. In US 4,460,706 werden keine Hinweise auf die kristalline Phasenzusammensetzung der Katalysatoren gegeben.

Das Dokument US 6,551,958 offenbart Dehydrier-Katalysatoren mit 50 bis 90 Gew.-% $Fe_2O_3$, 1 bis 40 Gew.-% $K_2O$, 5 bis 20 Gew.-% $Ce_2O_3$, 0,1 bis 10 Gew.-% MgO und 1 bis 10 Gew.-% CaO. Der Katalysator gemäß US 6,551,958 kann mehreren Mischoxide der Summenformel $K_2O\cdot(Fe_2O_3)_n$ enthalten, wobei n eine natürliche Zahl zwischen 1 und 11 ist. Es wird beschrieben, dass die Bildung der K/Fe-Mischoxide Kalzinierungstemperaturen über 750 °C erfordert. Für die Herstellung solcher Katalysatoren wird alpha-$Fe_2O_3$ mit einer speziellen Partikelgröße eingesetzt. US 6,551,958 beschreibt einen bevorzugten Temperaturbereich für die Kalzinierung von 800 bis 900°C. Die höhere Aktivität der erfindungsgemäßen Katalysatoren wird mit dem Gehalt an Kalium-Eisen-Mischoxid im Katalysator in Verbindung gebracht. In US 6,551,958 finden sich weder quantitativen Angaben zum Anteil der K/Fe-Mischoxide im fertigen Katalysator noch ein Hinweis auf das Vorliegen von Cerdioxid-Kristalliten definierter Größe.

Das Dokument EP 0 894 528 beschreibt Dehydrier-Katalysatoren enthaltend Eisenoxid, Kaliumoxid, Magnesium- und/oder Calciumoxide, Ceroxide, Wolfram- und/oder Molybdänoxide und Kaliumferrat. Es wird ein zweistufiges Verfahren zur Herstellung der Eisenoxid-basierten Dehydrier-Katalysatoren beschrieben, wobei ein möglichst hoher Anteil an Kaliumferraten beschrieben durch die Formel $K_2O \cdot nFe_2O_3$ erzeugt werden soll, welche in Form von Kristalliten kleiner als 2 μm vorliegen. In erster Stufe wird ein hydriertes Eisenoxid mit einer wässrigen Lösungen von Cernitrat imprägniert und mit wässriger Kaliumhydroxid-Lösung zu einer Paste verknetet, getrocknet und bei 600 bis 900 °C, 1 bis 6 h kalziniert. In der zweiten Stufe wird das kalzinierte Vorprodukt mit weitere Promotoren wie Molybdän-, Magnesium und Calciumverbindungen vermischt, zu Tabletten verarbeitet und bei 600 bis 900 °C, 1 bis 6 h endkalziniert. In den experimentellen Beispielen werden die beiden Kalzinierungsstufen bei 850 °C für 2 h und bei 650°C für 4 h durchgeführt. In EP-A 0 894 528 finden sich weder quantitativen Angaben zum Anteil der Kaliumferrat-Kristallite im fertigen Katalysator noch ein Hinweis auf das Vorliegen von Cerdioxid-Kristalliten definierter Größe.

Das Dokument EP-A 1 027 928 beschreibt Katalysatoren zur Dehydrierung von Ethylbenzol zu Styrol enthaltend ein spezielles Eisenoxid, das durch Sprühröstung einer Eisensalzlösung erhalten wurde. Die experimentellen Beispiele beschreiben Katalysatoren die verschiedenen Eisenoxide und weitere Metallkomponenten enthalten, wobei diese Katalysatoren bei 300 °C, 2h und bei 875 °C 1h kalziniert wurden. Es werden Cerdioxid-Kristallitgrößen von 23 bis 25 nm angegeben. In EP-A 1 027 928 findet sich kein Hinweis zum Einsatz oder zum Vorliegen einer Kalium-Eisen-Mischoxidphase im fertigen Katalysator.

Das Dokument WO 97/10898 beschreibt ein Verfahren zur Herstellung eines Dehydrierkatalysators enthaltend Eisen, Kalium und gegebenenfalls weitere Elemente, wobei in zwei Schritten bei unterschiedlichen Temperaturen kalziniert wird.

Die Publikationen Dulamita et al. (Applied Catalysis A: General, Bd. 287, Nr.1, 15.6.2005) und Liao et al. (Catalysis Communications, Bd. 9, Nr.9, 15.05.2008) beschreiben den Einfluss von Titandioxid-Promotoren auf das katalytische Verhalten von Eisenoxid/Kaliumoxid-Katalysatoren bei der Dehydrierung von Ethylbenzol zu Styrol. Das Dokument US-A 2013/165723 beschreibt einen Katalysator zur Dehydrierung von Kohlenwasserstoffen auf Basis von Eisenoxid, welcher zudem mindestens eine Kaliumverbindung, mindestens eine Cerverbindung, 0,7 bis 10 Gew.-% mindestens einer Manganverbindung und 10 bis 200 ppm mindestens einer Titanverbindung enthält, sowie ein Verfahren zu dessen Herstellung.

Es besteht ein Bedarf an weiter verbesserten Dehydrier-Katalysatoren für die Dehydrierung von Kohlenwasserstoffen, insbesondere für die Dehydrierung von Ethylbenzol, die eine verbesserte Katalysatoraktivität bei verbesserter oder gleichbleibender Stabilität und Standzeit aufweisen. Eine Aufgabe der vorliegenden Erfindung besteht darin, einen verbesserten Dehydrier-Katalysator auf der Basis von Eisenoxid bereitzustellen, der sich insbesondere durch eine verbesserte Katalysatoraktivität, insbesondere durch eine erhöhte Ausbeute auszeichnet. Ebenso sollen eine ausreichende mechanische Stabilität des Katalysators und eine Beständigkeit gegen Siedewasser gegeben sein.

Eine weitere Aufgabe der vorliegenden Erfindung besteht darin, Verfahren zur Herstellung der verbesserten Dehydrier-Katalysatoren bereitzustellen, welche einfach, kostengünstig und verlässlich durchzuführen sind, insbesondere sollten keine aufwändigen Verfahrensschritte, wie beispielsweise ein Sol-Gel-Verfahren, und/oder hohen Kalzinierungstemperaturen bei der Herstellung des Katalysators erforderlich sein.

Es wurde nun überraschenderweise gefunden, dass eine weitere Verbesserung der katalytischen Eigenschaften durch eine gezielte Optimierung der kristallinen Struktur der Dehydrier-Katalysatoren erreicht werden kann. Es wurde gefunden, dass optimierte Dehydrier-Ausbeuten, insbesondere Styrol-Ausbeuten, erhalten werden können, wenn die Katalysatoren einen hohen Gehalt an K/Fe-Mischoxidphasen von mindestens 20 Gew.-% und gleichzeitig Cerdioxid-Kristallite mit geringer Kristallitgröße im Bereich von 10 bis 30 nm aufweisen. Es konnte gezeigt werden, dass eine Kombination beider struktureller Merkmale erforderlich ist, um hohen Katalysatoraktivitäten, bei gleichzeitig guter mechanischer und chemischer Stabilität zu gewährleisten.

Es wurde zudem gefunden, dass sich die erfindungsgemäß optimierte kristalline Struktur des Katalysators beispielsweise durch eine gezielte Einstellung der Kalzinierungsbedingungen, wie Kalzinierungstemperatur und -dauer erzielen lässt.

Gegenstand der Beschreibung ist ein Dehydrier-Katalysator enthaltend mindestens eine Eisenverbindung, mindestens eine Kaliumverbindung und mindestens eine Cerverbindung, wobei die mindestens eine Eisenverbindung und die mindestens eine Kaliumverbindung zumindest teilweise in Form von einer oder mehreren K/Fe-Mischoxidphasen der allgemeinen Formel $K_x\text{-}Fe_yO_z$ vorliegen, wobei x eine Zahl von 1 bis 17 ist; y eine Zahl von 1 bis 22 ist, und z eine Zahl von 2 bis 34 ist, wobei der Katalysator mindestens 20 Gew.-%, bevorzugt mindestens 30 Gew.-%, insbesondere bevorzugt mindestens 40 Gew.-%, besonders bevorzugt mindestens 60 Gew.-%, bezogen auf den gesamten Katalysator, der K/Fe-Mischoxidphasen enthält und wobei der Katalysator kristallines Cerdioxid enthält, das eine Kristallitgröße im Bereich von 10 nm bis 30 nm, bevorzugt von 12 nm bis 25 nm, bevorzugt von 12 nm bis 22 nm, besonders bevorzugt von 14 nm bis 24 nm, besonders bevorzugt von 14 nm bis 22 nm, ganz besonders bevorzugt von 16 nm bis 19 nm, aufweist.

Gegenstand der Erfindung ist ein Dehydrier-Katalysator enthaltend

50 bis 90 Gew.-% mindestens einer Eisenverbindung, gerechnet als $Fe_2O_3$;

1 bis 30 Gew.-% mindestens einer Kaliumverbindung, gerechnet als $K_2O$;

2 bis 25 Gew.-% mindestens einer Cerverbindung, gerechnet als $CeO_2$;

0,1 bis 10 Gew.-% mindestens einer Magnesiumverbindung, gerechnet als MgO;

0,1 bis 10 Gew.-% mindestens einer Calciumverbindung, gerechnet als CaO;

0,1 bis 10 Gew.-% mindestens einer Molybdänverbindung, gerechnet als $MoO_3$;

1 bis 1.000 ppm mindestens einer Titanverbindung, gerechnet als $TiO_2$; und

0 bis 30 Gew.-% mindestens einer weiteren Komponente, wobei die mindestens eine Eisenverbindung und die mindestens eine Kaliumverbindung zumindest teilweise in Form von einer oder mehreren K/Fe-Mischoxidphasen der allgemeinen Formel $K_xFe_yO_z$ vorliegen, wobei x eine Zahl von 1 bis 17 ist; y eine Zahl von 1 bis 22 ist, und z eine Zahl von 2 bis 34 ist, wobei der Katalysator mindestens 60 Gew.-%, bezogen auf den gesamten Katalysator, der einen oder mehreren K/Fe-Mischoxidphasen enthält und wobei der Katalysator kristallines Cerdioxid enthält, das eine Kristallitgröße im Bereich von 12 nm bis 22 nm, besonders bevorzugt von 14 nm bis 22 nm, ganz besonders bevorzugt von 16 nm bis 19 nm, aufweist, wobei der Anteil an K/Fe-Mischoxidphase und die Kristallitgröße mittels Röntgendiffraktometrie bestimmt werden.

Soweit nicht anders angegeben, beziehen sich alle folgenden Angaben in Gew.-% auf den gesamten Dehydrier-Katalysator und sind jeweils gerechnet auf das Metalloxid in der höchsten Oxidationsstufe. Im Sinne der vorliegenden Erfindung ist ppm als Milligramm pro Kilogramm (mg/kg) zu verstehen.

Die erfindungsgemäßen Katalysatoren zeichnen sich durch eine verbesserte Aktivität aus im Vergleich zu den im Stand der Technik beschriebenen Katalysatoren.

Dehydrier-Katalysator enthaltend mindestens eine Eisenverbindung, mindestens eine Kaliumverbindung, mindestens eine Cerverbindung und optional weitere Metallverbindungen ist im Sinne der vorliegenden Erfindung so zu verstehen, dass die entsprechenden Metalle in den gegebenenfalls angegebenen Mengen im Katalysator bestimmt werden können. Im Katalysator können typischerweise Mischphasen (z.B. Oxid-Mischphasen) und/oder isolierte Phasen der im Folgenden beschriebenen Metallverbindungen vorliegen. Es ist zudem möglich, dass eine der mehrere der im Folgenden beschriebenen Komponente(n) teilweise oder vollständig in einem anderen bei der Katalysator-Herstellung verwendeten Rohstoff enthalten ist/sind. Erfindungsgemäß enthält der Dehydrier-Katalysator mindestens eine Eisenverbindung bzw. wird bei der Herstellung des Dehydrier-Katalysators mindestens eine Eisenverbindung eingesetzt. Bevorzugt ist die mindestens eine Eisenverbindung ausgewählt aus natürlichen oder synthetischen Eisenoxiden und/oder Eisenoxidhydroxiden. Insbesondere ist die mindestens eine Eisenverbindung ausgewählt aus der Gruppe bestehend aus $\alpha$-$Fe_2O_3$ (Hämatit), $\gamma$-$Fe_2O_3$, Eisenoxidhydroxid (z.B. $\alpha$-FeOOH, Goethit) und $Fe_3O_4$ (Magnetit). Als synthetische Eisenoxide können beispielsweise Eisenoxide eingesetzt werden, die durch thermische Zersetzung von Eisensalzlösungen hergestellt wurden.

Bevorzugt wird als Eisenverbindung ein Eisenoxid, insbesondere $Fe_2O_3$, bevorzugt $\alpha$-$Fe_2O_3$ (Hämatit) eingesetzt. Bevorzugt ist auch der Einsatz von $\alpha$-$Fe_2O_3$ (Hämatit) in Kombination mit Goethit (FeOOH) und/oder Magnetit ($Fe_3O_4$) als Eisenverbindung. Der Anteil an Goethit (FeOOH) und/oder Magnetit ($Fe_3O_4$) beträgt dann typischerweise 0 bis 30 Gew.-%, bezogen auf die Gesamtmenge der Eisenverbindungen.

Die spezifische Oberfläche der Eisenverbindung (z.B. mittels BET-Methode bestimmt) liegt typischerweise im Bereich von 1 bis 50 $m^2$/g, bevorzugt von 1 bis 20 $m^2$/g.

Die mindestens eine Eisenverbindung ist erfindungsgemäß in einer Menge im Bereich von 50 bis 90 Gew.-%, bevorzugt 60 bis 80 Gew.-%, besonders bevorzugt von 65 bis 75 Gew.-%, gerechnet als $Fe_2O_3$, im Dehydrier-Katalysator enthalten (bezogen auf das Gesamtgewicht des Dehydrier-Katalysators).

Erfindungsgemäß enthält der Katalysator mindestens eine Kaliumverbindung bzw. wird bei der Herstellung des Dehydrier-Katalysators mindestens eine Kaliumverbindung eingesetzt. Die mindestens eine Kaliumverbindung ist bevorzugt ausgewählt aus Kaliumoxid, Kaliumcarbonat, Kaliumhydroxid, Kaliumhydrogencarbonat, Kaliumoxalat und K/Fe-Mischoxiden, insbesondere ausgewählt aus Kaliumoxid, Kaliumcarbonat, Kaliumhydroxid und Kaliumhydrogencarbonat. Die mindestens eine Kaliumverbindung ist insbesondere ein Kaliumoxid ($K_2O$) oder ein Mischoxid. Es kann auch eine andere in der Wärme zersetzbaren Kaliumverbindung verwendet werden. Die mindestens eine Kaliumverbindung kann in dem Katalysator typischerweise als Oxid-Mischphase mit den im Katalysator vorhandenen Metallen vorliegen.

Die mindestens eine Kaliumverbindung ist erfindungsgemäß in einer Menge im Bereich von 1 bis 30 Gew.-%, bevorzugt von 5 bis 25 Gew.-%, insbesondere bevorzugt von 10 bis 15 Gew.-%, bezogen auf das Gesamtgewicht des Dehydrier-Katalysators und gerechnet als $K_2O$, im Dehydrier-Katalysator enthalten.

Das Kalium liegt bevorzugt in Form von einer oder mehreren unten beschriebenen K/Fe-Mischoxidphasen, in Form von Mischoxiden mit anderen Katalysatorkomponenten, insbesondere mit anderen Promotoren, beispielsweise Kaliummolybdat, Kaliumwolframat, Kaliumvanadat, Kaliummanganat, und/oder in Form von Kalium-Phasen, beispielsweise Kaliumoxid, im fertigen Katalysator vor.

Erfindungsgemäß liegt die mindestens eine Eisenverbindung und die mindestens eine Kaliumverbindung zumindest teilweise in Form von einer oder mehreren K/Fe-Mischoxidphasen der allgemeinen Formel $K_xFe_yO_z$ vor, wobei x eine Zahl von 1 bis 17 ist; y eine Zahl von 1 bis 22 ist, und z eine Zahl von 2 bis 34 ist. Oftmals werden diese K/Fe-Mischoxide bzw. K/Fe-Mischoxidphasen als Kaliumferrite bzw. Kaliumferritphasen oder als Kaliumferrate bzw. Kaliumferratphasen bezeichnet.

Unter K/Fe-Mischoxidphasen versteht man im Sinne der vorliegenden Erfindung Kalium-Eisen-Mischoxide der Summenformel $K_2O \cdot n\ Fe_2O_3$, wobei n eine Zahl von 0,1 bis 11 ist. Die Kalium-Eisen-Mischoxide können auch in der Form $K_aFe_bO_c$ beschrieben werden, wobei a, b und c natürliche Zahlen sind mit a = 1-17, b = 1-22 und c = 2-34. Bei den K/Fe-Mischoxidphasen kann es sich insbesondere um mindestens eine Verbindungen ausgewählt aus der Gruppe bestehend aus $KFeO_2$ , $K_2Fe_2O_4$ ($K_2O \cdot Fe_2O_3$) (mit n = 1); $K_2Fe_8O_{13}$ ($K_2O \cdot 4\ Fe_2O_3$) (mit n=4); $K_2Fe_{10}O_{16}$ ($K_2O \cdot 5\ Fe_2O_3$) (mit n = 5), $K_2Fe_{22}O_{34}$ ($K_2O \cdot 11\ Fe_2O_3$) (mit n = 11), $K_6Fe_2O_5$, $K_6Fe_2O_6$, $K_9(FeO_4)_2$ (Raumgruppe C2/c) und $K_{17}Fe_5O_{16}$ (Raumgruppe Cm) handeln. Bevorzugt handelt es sich bei der K/Fe-Mischoxidphase um mindestens eine Verbindungen ausgewählt aus der Gruppe bestehend aus $K_2Fe_2O_4$ ($K_2O \cdot Fe_2O_3$) (mit n = 1); $K_2Fe_8O_{13}$ ($K_2O \cdot 4\ Fe_2O_3$) (mit n=4); $K_2Fe_{10}O_{16}$ ($K_2O \cdot 5\ Fe_2O_3$) (mit n = 5) und $K_2Fe_{22}O_{34}$ ($K_2O \cdot 11\ Fe_2O_3$) (mit n = 11).

Die oben beschriebenen K/Fe-Mischoxide und Mischungen davon können durch die allgemeine Formel $K_xFe_yO_z$, beschrieben werden, wobei x eine Zahl zwischen 1 und 17 ist, y eine Zahl zwischen 1 und 22 ist und z eine Zahl zwischen 2 und 34 ist.

K/Fe-Mischoxidphasen können durch die Reaktion einer Eisenverbindung, z.B. Eisenoxid, Eisenhydroxid, Eisenoxihydroxid oder andere Eisensalzen, und einer Kaliumverbindung, z.B. Kaliumoxid, Kaliumhydroxid oder anderen Kaliumsalzen, bei höheren Temperaturen entstehen. Die kristalline Zusammensetzung des Katalysators kann röntgenographisch qualitativ und quantitativ bestimmt werden.

Erfindungsgemäß enthält der Dehydrier-Katalysator mindestens 60 Gew.-%, bezogen auf den gesamten Katalysator, der oben beschriebenen einen oder mehreren K/Fe-Mischoxidphasen. Bevorzugt enthält der Katalysator 60 bis 85 Gew.-%, bezogen auf den gesamten Katalysator, der einen oder mehreren K/Fe-Mischoxidphasen.

Bevorzugt sind Dehydrier-Katalysatoren, bei denen der überwiegende Teil des bei der Herstellung eingesetzten Eisens in Form von einer oder mehreren K/Fe-Mischoxidphasen der allgemeinen Formel $K_xFe_yO_z$, wie oben beschrieben, vorliegt. Es ist möglich, dass ein gewisser Anteil des Eisens in Form von anderen Eisenoxiden vorliegt, beispielsweise in Form von Eisenoxid-Phasen wie FeOOH, $Fe_2O_3$, insbesondere $\alpha$-$Fe_2O_3$ (Hämatit), und $Fe_3O_4$, (Magnetit). Bevorzugt kann ein Anteil des eingesetzten Eisens als Hämatit ($\alpha$-$Fe_2O_3$) im fertigen Dehydrier-Katalysator vorliegen.

Insbesondere liegen mindestens 50 Gew.-%, bevorzugt mindestens 60 Gew.-%, insbesondere bevorzugt mindestens 80 Gew.-% des Eisens, bezogen auf die gesamte Menge des eingesetzten Eisens, gerechnet als $Fe_2O_3$, in Form von einer oder mehreren der oben beschriebenen K/Fe-Mischoxidphasen der allgemeinen Formel $K_xFe_yO_z$ vor. Insbesondere können 0 bis 50 Gew.-%, bevorzugt 0 bis 40 Gew.-%, insbesondere bevorzugt 0 bis 20 Gew.-% des eingesetzten Eisens, gerechnet als $Fe_2O_3$, in Form einer Hämatit-Phase ($\alpha$(alpha)-$Fe_2O_3$) vorliegen. Erfindungsgemäß enthält der Dehydrier-Katalysator mindestens eine Cerverbindung bzw. wird bei der Herstellung des Dehydrier-Katalysators mindestens eine Cerverbindung eingesetzt. Die mindestens eine Cerverbindung ist bevorzugt ausgewählt aus Ceroxiden, Cerhydroxiden, Cercarbonaten, wasserhaltigen Cercarbonaten und Ceroxalaten. Bevorzugt können Mischungen der genannten Cerverbindungen eingesetzt werden. Bevorzugt ist die mindestens eine Cerverbindung ausgewählt aus Cer(IV)-oxid ($CeO_2$), Cer(III)-oxid ($Ce_2O_3$), Cer(III)-oxalat und Cer(III)-carbonat, bevorzugt aus Cer(IV)-oxid ($CeO_2$) und Cer(III)-carbonat. Typischerweise wird die mindestens eine Cerverbindung bei der Herstellung des Katalysators in Cerdioxid, insbesondere in kristallines Cerdioxid, umgewandelt.

Erfindungsgemäß enthält der Dehydrier-Katalysator 2 bis 25 Gew.-%, bevorzugt 5 bis 20 Gew.-%, bevorzugt 7 bis 20 Gew.-%, insbesondere bevorzugt 10 bis 20 Gew.-%, mindestens einer Cerverbindung, gerechnet als $CeO_2$.

Erfindungsgemäß enthält der Katalysator kristallines Cerdioxid, das eine Kristallitgröße im Bereich von 12 nm bis 22 nm, besonders bevorzugt von 14 nm bis 22 nm, ganz besonders bevorzugt von 16 nm bis 19 nm, aufweist. Insbesondere liegen mindestens 50 Gew.-%, bevorzugt mindestens 60 Gew.-%, bevorzugt mindestens 80 Gew.-%, insbesondere bevorzugt mindestens 90 Gew.-% des Cers, bezogen auf die gesamte Menge des eingesetzten Cers, gerechnet als $CeO_2$, in Form des oben beschriebenen kristallinen Cerdioxids mit der beschriebenen Kristallitgröße vor.

Kristallit im Sinne der vorliegenden Erfindung bezeichnet Einzelkristalle (auch Einzelkristall-Körner genannt) in einem polykristallinen Material, wie beispielsweise dem Katalysatormaterial, wobei diese Einzelkristalle in einer amorphen, teilkristallinen oder kristallinen Matrix eingebettet sein können und/oder neben Einzelkristallen anderer Art vorliegen können. Insbesondere sind Kristallite in einem polykristallinen Material durch ihre Korngrenzen voneinander und/oder zur Matrix abgegrenzt. Die Kristallite, bilden gegebenenfalls zusammen mit der umgebenen Matrix ein polykristallines Material, wobei verschiedenartige Kristallite im polykristallinen Material vorliegen können.

Kristallitgröße bedeutet im Sinne der vorliegenden Erfindung die mittlere Größe der Kristallite, bzw. der Kristallit-Körner, welche im Katalysator als polykristallines Material vorliegen. Insbesondere bezeichnet die Kristallitgröße die mittlere Größe der Kristallite im Katalysator, welche mittels Röntgenstrukturanalyse (Röntgendiffraktometrie XRD) und Auswertung der Peakbreite mittels der Scherrer-Gleichung erhalten werden kann. Die so bestimmte Kristallitgröße gibt Aufschluss über die Ausdehnung der kristallinen Körner im Katalysatormaterial, wobei Agglomerate der Körner bzw.

Kristallite nicht erfasst werden.

Cer ist überwiegend in Form von Cerdioxid, insbesondere als kristallines Cerdioxid, insbesondere als kristallines Cerdioxid in Form von Cerianit, im erfindungsgemäßen Katalysator enthalten. Das Cer kann gegebenenfalls zumindest teilweise auch als Mischverbindungen, insbesondere als Mischoxid, mit anderen Katalysatorkomponenten, im Katalysator vorliegen.

Der erfindungsgemäße Katalysator enthält Cerdioxid-Kristallite, welche im Wesentlichen aus kristallinem Cerdioxid, bevorzugt aus kristallinem Cerdioxid in Form von Cerianit, bestehen und welche als fein disperse Kristallit-Körner mit einem mittleren Durchmesser von 12 nm bis 22 nm, besonders bevorzugt von 14 nm bis 22 nm, ganz besonders bevorzugt von 16 nm bis 19 nm, vorliegen.

Erfindungsgemäß enthält der Dehydrier-Katalysator als weitere Komponenten mindestens eine Erdalkalimetallverbindung, bzw. es wird bei der Herstellung des Dehydrier-Katalysators mindestens eine Erdalkalimetallverbindung eingesetzt. Insbesondere kann der Dehydrier-Katalysator als weitere Komponente 0,1 bis 20 Gew.-%, insbesondere 0,1 bis 10 Gew.-%, bevorzugt 1 bis 5 Gew.-% mindestens einer Erdalkalimetallverbindung, gerechnet als Oxid, enthalten.

Erfindungsgemäß enthält der Dehydrier-Katalysator mindestens eine Magnesiumverbindung als weitere Komponente. Erfindungsgemäß enthält der Dehydrier-Katalysator als weitere Komponente 0,1 bis 10 Gew.-%, bevorzugt 1 bis 5 Gew.-%, mindestens einer Magnesiumverbindung, gerechnet als MgO. Insbesondere ist die mindestens eine Magnesiumverbindung ausgewählt aus Magnesiumoxid, Magnesiumcarbonat und Magnesiumhydroxid. Bevorzugt handelt es sich bei der mindestens einen Magnesiumverbindung um Magnesiumoxid (MgO) und/oder Magnesiumcarbonat ($MgCO_3$). Bevorzugt wird bei der Herstellung des Katalysators als weitere Komponente Magnesiumoxid (MgO) und/oder Magnesiumcarbonat ($MgCO_3$) eingesetzt.

Erfindungsgemäß enthält der Dehydrier-Katalysator mindestens eine Calciumverbindung als weitere Komponente. Erfindungsgemäß enthält der Dehydrier-Katalysator als weitere Komponente 0,1 bis 10 Gew.-%, bevorzugt 1 bis 5 Gew.-% mindestens einer Calciumverbindung, gerechnet als CaO. Insbesondere ist die mindestens eine Calciumverbindung ausgewählt aus Calciumoxid, Calciumcarbonat und Calciumhydroxid. Bevorzugt handelt es sich bei der mindestens einen Calciumverbindung um Calciumoxid (CaO). Bevorzugt wird bei der Herstellung des Katalysators als weitere Komponente Calciumoxid (CaO) und/oder Calciumhydroxid ($Ca(OH)_2$) eingesetzt.

Erfindungsgemäß enthält der Dehydrier-Katalysator mindestens eine Magnesiumverbindung und mindestens eine Calciumverbindung. Erfindungsgemäß enthält der Dehydrier-Katalysator 0,1 bis 10 Gew.-%, bevorzugt 1 bis 5 Gew.-% mindestens einer Magnesiumverbindung, gerechnet als MgO, sowie 0,1 bis 10 Gew.-%, bevorzugt 1 bis 5 Gew.-% mindestens einer Calciumverbindung, gerechnet als CaO.

Eine bevorzugte Ausführungsform der Beschreibung betrifft einen Dehydrier-Katalysator enthaltend

50 bis 90 Gew.-%, bevorzugt 60 bis 80 Gew.-%, mindestens einer Eisenverbindung, gerechnet als $Fe_2O_3$;

1 bis 30 Gew.-%, bevorzugt 5 bis 25 Gew.-%, mindestens einer Kaliumverbindung, gerechnet als $K_2O$;

2 bis 25 Gew.-%, bevorzugt 7 bis 20 Gew.-%, bevorzugt 10 bis 20 Gew.-%, mindestens einer Cerverbindung, gerechnet als $CeO_2$;

0,1 bis 10 Gew.-%, bevorzugt 0,1 bis 5 Gew.-%, mindestens einer Magnesiumverbindung, gerechnet als MgO;

0,1 bis 10 Gew.-%, bevorzugt 0,1 bis 5 Gew.-%, mindestens einer Calciumverbindung, gerechnet als CaO; und

0 bis 30 Gew.-%, bevorzugt 0,0001 bis 10 Gew.-%, mindestens einer weiteren Komponente.

In einer bevorzugten Ausführungsform addieren sich die oben genannten Komponenten zu 100 Gew.-%.

Die weiteren Komponenten können in Mengen von 0 bis 30 Gew.-%, bevorzugt von 0 bis 20 Gew.-%, bevorzugt von 0,0001 bis 10 Gew.-%, insbesondere bevorzugt von 0,001 bis 5 Gew.-%, insbesondere bevorzugt von 0,5 bis 5 Gew.-% enthalten sein (bzw. zugesetzt werden).

Typischerweise kann der Dehydrier-Katalysator als mindestens eine weitere Komponente, insbesondere als Promotor oder Dotierstoff, eine oder mehrere der üblichen Verbindungen zur Steigerung der Aktivität und/oder Selektivität enthalten. Beispielsweise kann als weitere Komponente mindestens eine Verbindung ausgewählt aus Verbindungen umfassend ein Metall ausgewählt aus der Gruppe bestehend aus Mn, Ti, Cr, Co, Ni, Cu, Zn, Al, Ga, Ge, Zr, Nb, Ru, Rh, Pd, Ag, Cd, In, Sn, Sb, La, Hf, Ta, Re, Ir, Pt, Au, Pb und Bi enthalten sein, bzw. es wird bei der Herstellung des Dehydrier-Katalysators mindestens eine solche Verbindung eingesetzt. Es ist weiterhin möglich, dass eine oder mehrere der oben genannten Komponenten bereits teilweise oder vollständig in einem anderen Rohstoff enthalten sind, der bei der Katalysatorherstellung verwendetet wird, beispielsweise im Eisenoxid. Die genannten üblichen Komponenten, insbesondere Promotoren oder Dotierstoffe, können typischerweise in Mengen von 0 bis 10 Gew.-%, vorzugsweise von 0,0001 bis 5 Gew.-%, vorzugsweise von 0,001 bis 2 Gew.-%, gerechnet jeweils als Oxid in der höchsten Oxidationsstufe, bezogen auf den gesamten Katalysator, enthalten sein.

Bevorzugt kann der Dehydrier-Katalysator als weitere Komponente mindestens eine Verbindung ausgewählt aus Verbindungen umfassend ein Metall ausgewählt aus der Gruppe bestehend aus Mangan (Mn), Molybdän (Mo), Titan (Ti), Vanadium (V) und Wolfram (W),), bevorzugt ausgewählt aus der Gruppe bestehend aus Molybdän (Mo), Titan (Ti), Vanadium (V) und Wolfram (W), enthalten; bzw. es wird bei der Herstellung des Dehydrier-Katalysators mindestens eine solche Verbindung eingesetzt. Die weitere Komponente kann insbesondere ausgewählt werden aus Sauerstoffverbindungen, beispielsweise Oxiden, Oxidhydraten, Oxoverbindungen, von Mangan (Mn), Molybdän (Mo), Titan (Ti), Vanadium (V) und Wolfram (W). Insbesondere handelt es sich bei der mindestens eine Verbindung ausgewählt aus Verbindungen umfassend ein Metall ausgewählt aus der Gruppe bestehend aus Mangan (Mn), Molybdän (Mo), Titan (Ti), Vanadium (V) und Wolfram (W) um eine Verbindung, die unter Hitzeeinwirkung bei der Herstellung des Dehydrier-Katalysators zerfällt.

Bevorzugt enthält der Dehydrier-Katalysator als weitere Komponente 0,0001 bis 10 Gew.-%, bevorzugt 0,001 bis 5 Gew.-%, besonders bevorzugt von 0,5 bis 5 Gew.-%, mindestens einer Verbindung ausgewählt aus der Gruppe bestehend aus Mangan (Mn), Molybdän (Mo), Titan (Ti), Vanadium (V) und Wolfram (W), bevorzugt ausgewählt aus der Gruppe bestehend aus Molybdän (Mo), Titan (Ti), Vanadium (V) und Wolfram (W), gerechnet als Oxid in der jeweils höchsten Oxidationsstufe.

Bevorzugt enthält der Dehydrier-Katalysator als weitere Komponente 0,0001 bis 10 Gew.-%, bevorzugt 0,001 bis 5 Gew.-%, besonders bevorzugt von 0,5 bis 5 Gew.-%, mindestens einer Verbindung ausgewählt aus Verbindungen umfassend ein Metall ausgewählt aus der Gruppe bestehend aus Mangan (Mn), Molybdän (Mo), Titan (Ti), Vanadium (V) und Wolfram (W), bevorzugt ausgewählt aus der Gruppe bestehend aus Molybdän (Mo), Titan (Ti), Vanadium (V) und Wolfram (W), gerechnet als Oxid in der jeweils höchsten Oxidationsstufe.

Bevorzugt wird als mindestens eine weitere Komponente mindestens eine Molybdänverbindung eingesetzt ausgewählt aus Molybdänoxiden und Molybdaten (z.B. Ammoniummolybdat, Kaliummolybdat). Bevorzugt handelt es sich bei der mindestens einen Molybdänverbindung um Molybdänoxid.

Erfindungsgemäß enthält der Dehydrier-Katalysator als weitere Komponente 0,1 bis 10 Gew.-%, bevorzugt 0,5 bis 5 Gew.-% mindestens einer Molybdänverbindung, gerechnet als $MoO_3$.

Erfindungsgemäß enthält der Dehydrier-Katalysator als weitere Komponente mindestens eine Titanverbindung; bzw. es kann bei der Herstellung des Dehydrier-Katalysators mindestens eine Titanverbindung eingesetzt werden. Der Dehydrier-Katalysator enthält 1 bis 1.000 ppm, bevorzugt 10 bis 500 ppm, bevorzugt 30 bis 500 ppm, besonders bevorzugt 50 bis 220 ppm, der mindestens einen Titanverbindung, gerechnet als $TiO_2$, bezogen auf den gesamten Katalysator.

Die mindestens eine Titanverbindung kann insbesondere ausgewählt sein aus Titanoxiden, Titanalkoholaten und Titancarboxylaten. Bevorzugt ist die mindestens eine Titanverbindung Titandioxid ($TiO_2$). Die mindestens eine Titanverbindung wird bei der Herstellung des Katalysators bevorzugt als Titandioxid ($TiO_2$) zugegeben. Es ist aber auch möglich andere Titanverbindungen einzusetzen. Es ist weiterhin möglich, dass die mindestens eine Titanverbindung bereits teilweise oder vollständig in einem anderen bei der Katalysatorherstellung verwendeten Rohstoff enthalten ist, z.B. im Eisenoxid.

Insbesondere bevorzugt enthält der Dehydrier-Katalysator als weitere Komponente 0,1 bis 10 Gew.-%, besonders bevorzugt 1 bis 5 Gew.-%, mindestens einer Vanadiumverbindung, gerechnet als $V_2O_5$.

In einer bevorzugten Ausführungsform bezieht sich die vorliegende Beschreibung auf einen Dehydrier-Katalysator wie oben beschrieben enthaltend:

50 bis 90 Gew.-%, bevorzugt 60 bis 80 Gew.-%, mindestens einer Eisenverbindung, gerechnet als $Fe_2O_3$;

1 bis 30 Gew.-%, bevorzugt 5 bis 25 Gew.-%, insbesondere bevorzugt 10 bis 20 Gew.-%, mindestens einer Kali-umverbindung, gerechnet als $K_2O$;

2 bis 25 Gew.-%, bevorzugt 5 bis 20 Gew.-%, bevorzugt 7 bis 20 Gew.-%, insbesondere bevorzugt 10 bis 20 Gew.-%, mindestens einer Cerverbindung, gerechnet als $CeO_2$;

0,1 bis 10 Gew.-%, bevorzugt 1 bis 5 Gew.-% mindestens einer Magnesiumverbindung gerechnet als MgO;

0,1 bis 10 Gew.-%, bevorzugt 1 bis 5 Gew.-%, mindestens einer Calciumverbindung, gerechnet als CaO; und

0,0001 bis 10 Gew.-%, bevorzugt 0,001 bis 5 Gew.-%, mindestens einer Verbindung ausgewählt aus Verbindungen umfassend ein Metall ausgewählt aus der Gruppe bestehend aus Mangan (Mn), Molybdän (Mo), Titan (Ti), Vanadium (V) und Wolfram (W), jeweils gerechnet als das Oxid in der höchsten Oxidationsstufe.

In einer bevorzugten Ausführungsform addieren sich die oben genannten Komponenten zu 100 Gew.-%.

Die vorliegende Erfindung bezieht sich auf einen Dehydrier-Katalysator wie oben beschrieben enthaltend:

50 bis 90 Gew.-%, bevorzugt 60 bis 80 Gew.-%, mindestens einer Eisenverbindung, gerechnet als $Fe_2O_3$;

1 bis 30 Gew.-%, bevorzugt 5 bis 25 Gew.-%, insbesondere bevorzugt 10 bis 20 Gew.-%, mindestens einer Kaliumverbindung, gerechnet als $K_2O$;

2 bis 25 Gew.-%, bevorzugt 5 bis 20 Gew.-%, bevorzugt 7 bis 20 Gew.-%, insbesondere bevorzugt 10 bis 20 Gew.-%, mindestens einer Cerverbindung, gerechnet als $CeO_2$;

0,1 bis 10 Gew.-%, bevorzugt 0,1 bis 5 Gew.-%, insbesondere bevorzugt 1 bis 4 Gew.-%, mindestens einer Magnesiumverbindung, gerechnet als MgO;

0,1 bis 10 Gew.-%, bevorzugt 0,1 bis 5 Gew.-%, insbesondere bevorzugt 1 bis 4 Gew.-%, mindestens einer Calciumverbindung, gerechnet als CaO;

0,1 bis 10 Gew.-%, bevorzugt 0,1 bis 5 Gew.-%, insbesondere bevorzugt 0,5 bis 4 Gew.-%, mindestens einer Molybdänverbindung, gerechnet als $MoO_3$ und

1 bis 1.000 ppm, bevorzugt 10 bis 500 ppm, bevorzugt 30 bis 500 ppm, insbesondere bevorzugt 50 bis 220 ppm, mindestens einer Titanverbindung, gerechnet als $TiO_2$

In einer bevorzugten Ausführungsform addieren sich die oben genannten Komponenten zu 100 Gew.-%.

In einer bevorzugten Ausführungsform bezieht sich die vorliegende Erfindung auf einen Dehydrier-Katalysator wie oben beschrieben enthaltend:

50 bis 90 Gew.-%, bevorzugt 60 bis 80 Gew.-%, mindestens einer Eisenverbindung, gerechnet als $Fe_2O_3$;

1 bis 30 Gew.-%, bevorzugt 5 bis 25 Gew.-%, insbesondere bevorzugt 10 bis 20 Gew.-%, mindestens einer Kaliumverbindung, gerechnet als $K_2O$;

2 bis 25 Gew.-%, bevorzugt 5 bis 20 Gew.-%, bevorzugt 7 bis 20 Gew.-%, insbesondere bevorzugt 10 bis 20 Gew.-%, mindestens einer Cerverbindung, gerechnet als $CeO_2$;

0,1 bis 10 Gew.-%, bevorzugt 0,1 bis 5 Gew.-%, insbesondere bevorzugt 1 bis 4 Gew.-%, mindestens einer Magnesiumverbindung, gerechnet als MgO;

0,1 bis 10 Gew.-%, bevorzugt 0,1 bis 5 Gew.-%, insbesondere bevorzugt 1 bis 4 Gew.-%, mindestens einer Calciumverbindung, gerechnet als CaO;

0,1 bis 10 Gew.-%, bevorzugt 0,1 bis 5 Gew.-%, insbesondere bevorzugt 0,5 bis 4 Gew.-%, mindestens einer Molybdänverbindung, gerechnet als $MoO_3$;

0,1 bis 20 Gew.-%, bevorzugt 0,1 bis 10 Gew.-%, bevorzugt 0,2 bis 5 Gew.-%, insbesondere bevorzugt 0,7 bis 2 Gew.-%, mindestens einer Manganverbindung, gerechnet als $MnO_2$; und

1 bis 1.000 ppm, bevorzugt 10 bis 500 ppm, bevorzugt 30 bis 500 ppm, insbesondere bevorzugt 50 bis 220 ppm, mindestens einer Titanverbindung, gerechnet als $TiO_2$

In einer bevorzugten Ausführungsform addieren sich die oben genannten Komponenten zu 100 Gew.-%.

Alle Angaben in Gew.-% beziehen sich - soweit nicht anders angegeben - auf den gesamten Dehydrier-Katalysator. Alle Angaben in Gew.-% wurden - soweit nicht anders angegeben - auf das Oxid des betreffenden Metalls jeweils in der höchsten Oxidationsstufe berechnet.

In einer Ausführungsform enthält der oben beschriebene Dehydrier-Katalysator als weitere Komponente mindestens eine weitere Seltenerdenmetallverbindung neben Cer, insbesondere ausgewählt aus der Gruppe bestehend aus Lanthan (La), Praseodym (Pr) und Neodym (Nd). Bevorzugt enthält der Dehydrier-Katalysator 1 bis 1.000 ppm, bevorzugt 10 bis 500 ppm, besonders bevorzugt 20 bis 300 ppm, mindestens einer weiteren Seltenerdenmetallverbindung, neben Cer, gerechnet als Oxid in der jeweils höchsten Oxidationsstufe. Insbesondere enthält der Katalysator 1 bis 1.000 ppm, bevorzugt 10 bis 500 ppm, besonders bevorzugt 20 bis 300 ppm, mindestens einer Seltenerdenmetallverbindung ausgewählt aus der Gruppe bestehend aus Lanthan, Praseodym und Neodym. Bevorzugt kann der Dehydrier-Katalysator als weitere Komponente 1 bis 1.000 ppm, bevorzugt 3 bis 500, besonders bevorzugt 10 bis 100 ppm mindestens einer Lanthanverbindung, gerechnet als $La_2O_3$, enthalten. Bevorzugt kann der Dehydrier-Katalysator als weitere Komponente 1 bis 1.000 ppm, bevorzugt 3 bis 500, besonders bevorzugt 10 bis 100 ppm mindestens einer Praseodymverbindung, gerechnet als $PrO_2$, enthalten. Bevorzugt kann der Dehydrier-Katalysator als weitere Komponente 1 bis 1.000 ppm, bevorzugt 3 bis 500, besonders bevorzugt 10 bis 100 ppm mindestens einer Neodymverbindung, gerechnet als $Nd_2O_3$, enthalten.

Der oben beschriebene Dehydrier-Katalysator kann vorzugsweise als weitere Komponente mindestens eine Verbindung von Metallen der 8. bis 12. Nebengruppe des Periodensystems enthalten. Bevorzugt enthält der oben beschriebene Dehydrier-Katalysator als weitere Komponente mindestens eine Verbindung von Metallen ausgewählt aus der Gruppe bestehend aus Mangan (Mn), Ruthenium (Ru), Osmium (Os), Kobalt (Co), Nickel (Ni), Palladium (Pd), Platin (Pt), Kupfer (Cu), Silber (Ag), Gold (Au) und Zink (Zn); bevorzugt ausgewählt aus der Gruppe bestehend aus Kobalt (Co), Mangan (Mn), Palladium (Pd), Kupfer (Cu), und Zink (Zn); besonders bevorzugt ausgewählt aus der Gruppe bestehend aus Mangan (Mn), Kupfer (Cu), und Zink (Zn). Der oben beschriebene Dehydrier-Katalysator kann als weitere Komponente insbesondere 1 bis 1.000 ppm, bevorzugt 50 bis 500 ppm, besonders bevorzugt 50 bis 200 ppm, mindestens einer Verbindung von Metallen der 8. bis 12. Nebengruppe des Periodensystems, gerechnet jeweils als Oxid in der höchsten Oxidationsstufe, enthalten. In einer bevorzugten Ausführungsform enthält der oben beschriebene Dehydrier-Katalysator 1 bis 1.000 ppm, bevorzugt 50 bis 500 ppm, besonders bevorzugt 50 bis 200 ppm, mindestens einer Verbindung von Metallen ausgewählt aus der Gruppe bestehend aus Mangan (Mn), Kupfer (Cu), und Zink (Zn), gerechnet jeweils als das Oxid in der höchsten Oxidationsstufe. Bevorzugt kann der Dehydrier-Katalysator als weitere Komponente 1 bis 1.000 ppm, bevorzugt 30 bis 500 ppm, besonders bevorzugt 30 bis 200 ppm, mindestens einer Manganverbindung, gerechnet als $MnO_2$, enthalten. Bevorzugt kann der Dehydrier-Katalysator als weitere Komponente 1 bis 1000 ppm, bevorzugt 10 bis 200, besonders bevorzugt 30 bis 100 ppm mindestens einer Kupferverbindung, gerechnet als CuO, enthalten. Bevorzugt kann der Dehydrier-Katalysator als weitere Komponente 1 bis 1.000 ppm, bevorzugt 1 bis 500 ppm, besonders bevorzugt 10 bis 100 ppm, mindestens einer Zinkverbindung, gerechnet als ZnO, enthalten.

Darüber hinaus kann der Dehydrier-Katalysator als weitere Komponente mindestens eine Verbindung von Elementen der 4. Hauptgruppe des Periodensystems der Elemente enthalten. Bevorzugt enthält der oben beschriebene Dehydrier-Katalysator als weitere Komponente mindestens eine Verbindung ausgewählt aus der Gruppe bestehend aus Silicium (Si)-, Germanium (Ge)-, Zinn (Sn)-, und Blei (Pb)-Verbindungen, bevorzugt mindestens eine Siliciumverbindung.

Insbesondere enthält der Dehydrier-Katalysator 1 bis 1.000 ppm, bevorzugt 5 bis 500 ppm, besonders bevorzugt 10 bis 100 ppm, mindestens einer Verbindung von ausgewählt aus der Gruppe bestehend aus Silicium (Si)-, Germanium (Ge)-, Zinn (Sn)-, und Blei (Pb)-Verbindungen, gerechnet als Oxid in der jeweils höchsten Oxidationsstufe. In einer Ausführungsform enthält der beschriebene Dehydrier-Katalysator 1 bis 1000 ppm, bevorzugt 5 bis 500 ppm, besonders bevorzugt 10 bis 100 ppm, mindestens einer Siliciumverbindung, gerechnet als $SiO_2$.

Typischerweise kann der oben beschriebene Dehydrier-Katalysator als Nichtmetall, neben Sauerstoff, mindestens ein Nicht-Metall ausgewählt aus Nichtmetallen der 5. bis 7. Hauptgruppe des Periodensystems enthalten, insbesondere ausgewählt aus der Gruppe bestehend aus Stickstoff, Phosphor, Schwefel und Chlor.

Der erfindungsgemäße Dehydrier-Katalysator kann insbesondere eine spezifische Oberfläche im Bereich von 1 bis 3 $m^2$/g, insbesondere von 1,5 bis 2,5 $m^2$/g aufweisen. Die spezifische Oberfläche der Katalysatoren kann beispielsweise mittels der Stickstoffadsorptionsmethode, z.B. wie in DIN ISO 9277 beschrieben, ermittelt werden.

Insbesondere bezieht sich die vorliegende Erfindung auf einen oben beschriebenen Dehydrier-Katalysator zur katalytischen Dehydrierung von Kohlenwasserstoffen bei einem molaren Dampf/Kohlenwasserstoff-Verhältnis 1 bis 20; bevorzugt im Bereich von 1 bis 10; insbesondere bevorzugt im Bereich von 1 bis 9, besonders bevorzugt von 5 bis 8,5.

In einem weiteren Aspekt betrifft die vorliegende Erfindung ein Verfahren zur Herstellung eines oben beschriebenen Dehydrier-Katalysators wie in Anspruch 3 beschrieben.

In einem weiteren Aspekt betrifft die vorliegende Beschreibung ein Verfahren zur Herstellung eines oben beschriebenen Dehydrier-Katalysators umfassend die folgenden Schritte:

i) Herstellen einer Katalysator-Vormischung durch Vermischen mindestens einer Eisenverbindung, mindestens einer Kaliumverbindung, mindestens einer Cerverbindung und optional weiterer Metallverbindungen, optional weiterer Komponenten und optional mindestens eines Bindemittels mit einem Lösungsmittel;

ii) Herstellen von Katalysator-Formkörpern aus der in Schritt i) erhaltenen Katalysator-Vormischung;

iii) Trocknen der Katalysator-Formkörper und Kalzinieren der Katalysator-Formkörper, wobei das Kalzinieren der Katalysator-Formkörper bei einer Temperatur im Bereich von 600 °C und 1.000 °C, bevorzugt von 700 °C bis 950 °C, bevorzugt von 750 °C bis 900 °C, insbesondere bevorzugt von 600 °C bis 850 °C und über einen Zeitraum von 10 min bis 300 min, bevorzugt von 15 min bis 240 min, erfolgt.

Für das Verfahren zur Herstellung eines Dehydrier-Katalysators werden bevorzugt die oben im Zusammenhang mit dem Dehydrier-Katalysator beschriebenen Eisenverbindungen, Kaliumverbindungen, Cerverbindungen und weitere Komponenten, insbesondere weitere Metallverbindungen, bevorzugt in den beschriebenen Mengen, eingesetzt. Optional können die oben beschriebenen Komponenten bei der Herstellung eingesetzt werden, optional befinden sich eine oder mehrere der Komponenten ganz oder teilweise in einem der eingesetzten Rohstoffe, beispielsweise im verwendeten Eisenoxid und/oder Cercarbonat.

Die grundlegende Vorgehensweise bei der Herstellung von Dehydrier-Katalysatoren ist dem Fachmann bekannt. Die Herstellung der oben beschriebenen Dehydrier-Katalysatoren kann beispielsweise wie in US 6,551,958 beschrieben erfolgen.

Die Auswahl der Katalysator-Komponenten (Edukte, Rohstoffe) und die Wahl der Herstellbedingungen, insbesondere der Kalzinierungsbedingungen können so erfolgen, dass erfindungsgemäße Katalysatoren mit der beschriebenen Kombination struktureller Merkmale erhalten werden. Besonders vorteilhaft ist ein Herstellverfahren, das möglichst wenige Schritte, insbesondere möglichst wenig Kalzinierungsschritte, aufweist.

Es wurde beispielsweise gefunden, dass die erfindungsgemäßen Katalysatoren, welche die beschriebene vorteilhafte Kombination an strukturellen Merkmalen (Anteil an K/Fe-Mischoxidphasen und $CeO_2$-Kristallitgröße) aufweisen, bevorzugt dadurch erhalten werden können, dass die Temperatur sowie die Dauer oder die Verweilzeit der Kalzinierung eingestellt und kontrolliert werden. K/Fe-Mischoxidphasen der allgemeinen Formel $K_xFe_yO_z$, wobei x eine Zahl von 1 bis 17 ist; y eine Zahl von 1 bis 22 ist, und z eine Zahl von 2 bis 34 ist, können beispielsweise durch die Reaktion der mindestens einen Eisenverbindung und der mindestens einen Kaliumverbindungen während der Endkalzinierung des Katalysators in Anwesenheit aller anderer Bestandteile des Katalysators entstehen.

Es wurde insbesondere gefunden, dass für die Bildung von K/Fe-Mischoxidphasen hohe Kalzinierungstemperaturen und lange Kalzinierungsdauern erforderlich sind, aber andererseits hohe Temperaturen und lange Kalzinierungsdauern zur Vergrößerung der Cerdioxid-Kristallitgröße führen, was beispielsweise darauf zurückgeführt werden kann, dass die Cerdioxid-Kristallite sintern und damit ihre Größe anwächst. Es wurden unter anderem optimale Kombinationen von Temperatur und Dauer des Kalzinierens in Schritt iii) gefunden. Grundsätzlich können die erfindungsgemäßen Katalysatoren erhalten werden, indem Schritt iii) entweder bei tieferen Kalzinierungstemperaturen über einen längeren Zeitraum oder bei höheren Kalzinierungstemperaturen über einen kürzeren Zeitraum in den angegebenen Grenzen durchgeführt wird.

Zur Herstellung der Katalysator-Vormischung werden in der Regel die Komponenten, typischerweise in Form von festen Pulvern, vermischt und dann mit einem Lösungsmittel, insbesondere Wasser, gegebenenfalls unter Zugabe eines Bindemittels vermischt. Das Vermischen erfolgt bevorzugt durch inniges Vermischen, z.B. durch Verkneten, in einem Rührkessel, Mixmuller, Mixer, Kneter oder Extruder, bevorzugt in einem Mixmuller, Kneter oder Mixer. Unter Lösungsmittel ist in diesem Zusammenhang insbesondere ein flüssiges Lösungs- und/oder Dispergiermittel zu verstehen, in dem die festen Katalysatorkomponenten dispergiert werden.

Als Lösungsmittel wird insbesondere Wasser oder eine Mischung aus Wasser und aus polaren Lösungsmitteln, z.B. Alkoholen, Estern, eingesetzt. Als Bindemittel (auch Plastifizierhilfsmittel) können beispielsweise Alginat, Stärke, Carboxymethylcellulose, Hydroxyethylcellulose und Polyvinylalkohol eingesetzt werden. Typischerweise werden die Bindemittel in Form einer Lösung in Wasser eingesetzt.

Aus der so erhaltenen Katalysator-Vormischung werden dann typischerweise Katalysator-Formkörper hergestellt, die anschließend getrocknet und kalziniert werden.

Die Herstellung von Katalysator-Formkörpern aus der Katalysator-Vormischung erfolgt typischerweise durch extrudieren oder pressen (Tablettenpressen). Beispiele für Katalysator-Formkörper sind Zylinder (Pellets), Ringe, Sternkörper und Wabenkörper. Bevorzugt erfolgt die Herstellung von Katalysator-Formkörpern aus der in Schritt i) erhaltenen Katalysator-Vormischung mittels Extrusion.

Nach der Formgebung werden die feuchten Formkörper typischerweise bei Temperaturen von 50 °C bis 500 °C, bevorzugt von 80 bis 350°C getrocknet. Die Trocknung kann beispielsweise im Trockenschank (z.B. auf Blechen), in einer Trocknungstrommel und/oder auf Bandtrockner stattfinden.

Anschließend werden die Formkörper in der Regel kalziniert. Das Kalzinieren kann in Muffelofen, Bandkalzinierer, Drehrohrofen usw. durchgeführt werden. Vorzugsweise werden die Katalysatorstränge in einem Drehrohrofen kalziniert.

Bevorzugt werden die Katalysator-Formkörpern in Schritt iii) bei Temperaturen im Bereich von 600 °C bis 1000 °C, bevorzugt von 700 °C bis 950 °C, bevorzugt von 750 °C bis 900 °C, insbesondere bevorzugt von 600 °C bis 850 °C kalziniert. Das Kalzinieren kann beispielsweise in einem Drehrohrofen durchgeführt werden.

In einer bevorzugten Ausführungsform betrifft die Beschreibung ein Verfahren zur Herstellung eines Dehydrier-Katalysators wie oben beschrieben, wobei das Kalzinieren der Katalysator-Formkörper in Schritt iii)

bei Temperaturen im Bereich von 600 °C bis 850 °C, bevorzugt von 750 °C bis 850 °C, besonders bevorzugt von 775 °C bis 825 °C, und über einen Zeitraum von 10 min bis 300 min, bevorzugt von 30 min bis 180 min, besonders bevorzugt von 30 min bis 90 min, ganz besonders bevorzugt von 30 min bis 60 min, oder

bei Temperaturen im Bereich von 800 °C bis 1.000 °C, bevorzugt von 850 °C bis 1.000 °C, bevorzugt von 850 °C bis 900 °C, und über einen Zeitraum von 10 min bis 60 min, bevorzugt von 10 min bis 50 min, bevorzugt von 10 min bis 30 min,

erfolgt.

Erfindungsgemäß kann das Kalzinieren in Schritt iii) bei Temperaturen von 700 °C bis 800 °C, bevorzugt von 750 °C bis 800 °C und bei einer Kalzinierungsdauer von 15 min bis 90 min, bevorzugt von 30 min bis 60 min, erfolgen, wobei das Verfahren als stationäres Verfahren, beispielsweise in einem Muffelofen, durchgeführt wird.

Erfindungsgemäß kann das Kalzinieren in Schritt iii) bei Temperaturen von 800 °C bis 950 °C, bevorzugt 850 °C bis 900 °C, und bei einer Kalzinierungsdauer von 10 min bis 30 min, bevorzugt von 10 min bis 20 min, erfolgen, wobei das Verfahren als kontinuierliches Verfahren, beispielsweise in einem kontinuierlichen Drehrohrofen, durchgeführt wird. Typischerweise entspricht in einem kontinuierlichen Verfahren die Verweilzeit der oben beschriebenen Kalzinierungsdauer.

Als Verbindungen und weitere Komponenten können Verbindungen eingesetzt werden, wie sie im fertigen Katalysator vorliegen, oder Verbindungen, die sich während des Herstellungsprozess in Verbindungen umwandeln, wie sie im fertigen Katalysator vorliegen. Es werden bevorzugt die im Zusammenhang mit dem erfindungsgemäßen Dehydrier-Katalysator beschriebenen Verbindungen eingesetzt.

Insbesondere betrifft die Erfindung ein Verfahren zur Herstellung eines Dehydrier-Katalysators wie beschrieben, wobei eine Eisenverbindung eingesetzt wird, die zu mindestens 50 Gew.-%, bevorzugt zu mindestens 80 Gew.-%, bezogen auf die gesamte Eisenverbindung, Eisen(III)-oxid ($Fe_2O_3$) enthält. Als Eisenkomponente wird überwiegend Hämatit ($Fe_2O_3$) eingesetzt. Ein Teil des Eisens kann als Goethit (FeOOH) oder Magnetit ($Fe_3O_4$) eingesetzt werden, wobei dieser Anteil 0 bis 30% betragen kann. Insbesondere betrifft die Erfindung ein Verfahren zur Herstellung eines Dehydrier-Katalysators wie beschrieben, wobei als Kaliumverbindung ein Kaliumoxid, Kaliumhydroxid, Kaliumcarbonat und/oder Kaliumhydrogencarbonat eingesetzt werden. Bevorzugt werden als Cerverbindung Ceroxide, insbesondere Cerdioxid, Cercarbonate, Cerhydroxide, und/oder Cerhydrogencarbonate eingesetzt. Bevorzugt werden als Magnesiumverbindung Magnesiumoxid, Magnesiumcarbonat und/oder Magnesiumhydroxid eingesetzt. Bevorzugt werden als Calciumverbindung Calciumoxid, Calciumcarbonat und/oder Calciumhydroxid eingesetzt. Bevorzugt werden als Titanverbindung Titanoxide, insbesondere $TiO_2$, Titanalkoholate und/oder Titancarboxylate eingesetzt. Bevorzugt werden als Molybdänverbindung Molybdänoxide, insbesondere $MoO_3$ eingesetzt. Bevorzugt werden als Vanadiumverbindung Vanadiumoxide eingesetzt.

In einem weiteren Aspekt betrifft die vorliegende Erfindung ein Verfahren zur katalytischen Dehydrierung eines Kohlenwasserstoffs, wobei ein Gemisch aus Wasserdampf und mindestens einem Kohlenwasserstoff mit einem oben beschriebenen Dehydrier-Katalysator in Kontakt gebracht wird.

Die oben im Zusammenhang mit dem erfindungsgemäßen Dehydrier-Katalysator und mit dem Verfahren zu seiner Herstellung beschriebenen bevorzugten Ausführungsformen gelten in analoger Weise für das erfindungsgemäße Verfahren zur katalytischen Dehydrierung eines Kohlenwasserstoffs.

Das Verfahren zur katalytischen Dehydrierung eines Kohlenwasserstoffs unter Verwendung des erfindungsgemäßen Dehydrier-Katalysator zeichnet sich durch eine verbesserte Ausbeute, beispielsweise eine verbesserte Styrol-Ausbeute, im Vergleich zu bekannten Verfahren bzw. Dehydrier-Katalysatoren, aus.

Bevorzugt betrifft die vorliegende Erfindung ein Verfahren zur katalytischen Dehydrierung eines Kohlenwasserstoffs, wobei ein Gemisch aus Wasserdampf und mindestens einem Kohlenwasserstoff mit einem molaren Dampf/Kohlenwasserstoff-Verhältnis im Bereich von 1 bis 20; bevorzugt von 1 bis 10; insbesondere bevorzugt von 1 bis 9; besonders bevorzugt von 5 bis 8,5; eingesetzt wird. Insbesondere betrifft die Erfindung ein Verfahren zur katalytischen Dehydrierung von Ethylbenzol zu Styrol, wobei ein Gemisch aus Wasserdampf und Ethylbenzol mit einem Dampf/Kohlenwasserstoff-Gewichtsverhältnis im Bereich von 0,17 bis 3,4; bevorzugt von 0,17 bis 1,7; insbesondere bevorzugt von 0,17 bis 1,5; besonders bevorzugt von 0,9 bis 1,45; eingesetzt wird. Insbesondere bevorzugt kann die erfindungsgemäße katalytische Dehydrierung eines Kohlenwasserstoffs mit einem mittleren Dampf/Kohlenwasserstoff-Gewichtsverhältnis im Bereich von 0,9 bis 1,45 (kg/kg) durchgeführt werden.

Typischerweise werden bei dem erfindungsgemäßen Verfahren zur katalytischen Dehydrierung pro Durchgang durch den Reaktor (per-pass) Ausbeuten von 40 bis 80 %, bevorzugt von 50 bis 75 %, besonders bevorzugt von 60 bis 70 %, bezogen auf den eingesetzten Kohlenwasserstoff erreicht. Insbesondere werden bei der katalytischen Dehydrierung von Ethylbenzol pro Durchgang durch den Reaktor Styrol-Ausbeuten von 40 bis 80 %, bevorzugt von 50 bis 75 %, besonders bevorzugt von 60 bis 70 %, bezogen auf das eingesetzten Ethylbenzol, erreicht. Die angegebenen Ausbeuten beziehen sich auf Mol-%.

Typischerweise wird das Verfahren zur katalytischen Dehydrierung eines Kohlenwasserstoffs bei Temperaturen von 500 bis 650 °C und Drücken von 0,2 bis 2 bar absolut durchgeführt.

Bei den beschriebenen Verfahren kann es sich um die Dehydrierung von alkylaromatischen oder aliphatischen Kohlenwasserstoffen handeln, bevorzugt handelt es sich um die Dehydrierung von alkylaromatischen Kohlenwasserstoffen, besonders bevorzugt um die Dehydrierung von Ethylbenzol zu Styrol. Bei den erfindungsgemäßen Verfahren zur Dehydrierung eines Kohlenwasserstoffs kann es sich beispielsweise um die Dehydrierung von Ethylbenzol zu Styrol, die Dehydrierung von Isopropylbenzol zu alpha-Methylstyrol, die Dehydrierung von Buten zu Butadien oder die Dehydrierung von Isoamylen zu Isopren handeln. Bevorzugt handelt es sich bei dem Kohlenwasserstoff um Ethylbenzol.

Weiterhin bezieht sich die vorliegende Erfindung auf die Verwendung eines oben beschriebenen Dehydrier-Katalysators zur katalytischen Dehydrierung eines Kohlenwasserstoffs, insbesondere eines alkylaromatischen oder aliphatischen Kohlenwasserstoffs, bevorzugt eines alkylaromatischen Kohlenwasserstoffs, besonders bevorzugt von Ethylbenzol. Bevorzugt bezieht sich die Erfindung auf die Verwendung eines oben beschriebenen Dehydrier-Katalysators zur katalytischen Dehydrierung eines Kohlenwasserstoffs bei einem molaren Dampf/Kohlenwasserstoff-Verhältnis im Bereich von 1 bis 20; bevorzugt im Bereich von 1 bis 10; insbesondere bevorzugt im Bereich von 1 bis 9; besonders bevorzugt im Bereich von 5 bis 8,5.

Im Folgenden werden die Figuren erläutert:

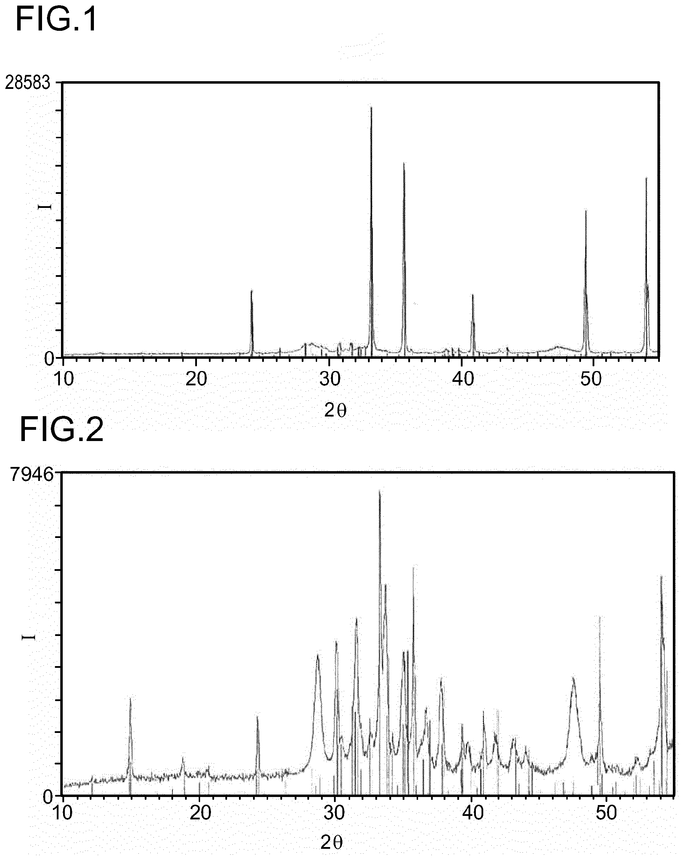

Fig. 1 zeigt das Röntgendiffraktogramm des Katalysators V1 gemäß Beispiel 1 (Vergleichsbeispiel). Das Röntgendiffraktogramm zeigt auf der y-Achse die Intensität I ($L_{in}$ (Counts)) und auf der x-Achse die 2$\Theta$ (2Theta)-Skala.

Fig. 2 zeigt das Röntgendiffraktogramm des Katalysators K5 gemäß Beispiel 5 (erfindungsgemäßes Beispiel). Das Röntgendiffraktogramm zeigt auf der y-Achse die Intensität I ($L_{in}$ (Counts)) und auf der x-Achse die 2$\Theta$ (2Theta)-Skala.

Die vorliegende Erfindung soll durch die folgenden Beispiele näher erläutert werden.

Beispiele

Beispiel 1 (Vergleichsbeispiel)

Als Komponenten wurden Eisenoxid (alpha-$Fe_2O_3$, Hämatit), Kaliumcarbonat ($K_2CO_3$), Cercarbonat ($Ce_2CO_3$), Magnesiumoxid (MgO), Molybdänoxid ($MoO_3$), Calciumhydroxid ($Ca(OH)_2$), Mangandioxid ($MnO_2$) und Titandioxid ($TiO_2$) eingesetzt.

Die oben genannten pulverförmigen Komponenten wurden zunächst trocken vermischt und dann unter Zugabe von Wasser und Stärkelösung geknetet. Die Katalysatormasse wurde extrudiert, wobei Pellets mit 3 mm Durchmesser erhalten wurden. Die Katalysator-Formkörper (Pellets) wurden 1 h bei 120 °C getrocknet, dann 1 h bei 350 °C und 1 h bei 500 °C in Luft in einem Muffelofen kalziniert.

Es wurde eine Katalysator K1 mit der folgenden nominalen Oxidzusammensetzung erhalten:

| | |
|---|---|
| 71,1 Gew.-% | $Fe_2O_3$, |
| 13,6 Gew.-% | $K_2O$, |
| 7,4 Gew.-% | $CeO_2$, |
| 2,2 Gew.-% | MgO, |
| 2,0 Gew.-% | CaO, |
| 2,1 Gew.-% | $MoO_3$, |
| 1,6 Gew.-% | $MnO_2$ und |
| 70 ppm | $TiO_2$ |

Beispiel 2 (Vergleichsbeispiel)

Es wurde einen Katalysator wie in Beispiel 1 beschrieben hergestellt, mit dem einzigen Unterschied, dass die Endkalzinierung 1 h bei 870 °C stattfand.

Es wurde der Katalysator K2 mit der oben genannten nominalen Zusammensetzung erhalten.

Beispiel 3 (Vergleichsbeispiel)

Es wurde einen Katalysator wie in Beispiel 1 beschrieben hergestellt, mit dem einzigen Unterschied, dass die Endkalzinierung 1 h bei 1000°C stattfand.

Es wurde der Katalysator K3 mit der oben genannten nominalen Zusammensetzung erhalten.

Beispiele 4 bis 8 (erfindungsgemäße Beispiele und Vergleichsbeispiele)

Es wurde einen Katalysator wie in Beispiel 1 beschrieben hergestellt, mit dem einzigen Unterschied, dass die Endkalzinierung bei verschiedenen Temperaturen im Bereich von 775 °C bis 825 °C und über verschiedene Zeiträume im Bereich von 30 min bis 60 min durchgeführt wurden. Das Kalzinieren erfolgte wie im Vergleichsbeispiel 1 in einem Muffelofen stationär in LuftAtmosphäre.

Es wurden die Katalysatoren K4 bis K8 mit der oben genannten nominalen Zusammensetzung erhalten.

Die Kalzinierungsbedingungen sind in der Tabelle 1 unten zusammengefasst.

Beispiel 9: Charakterisierung der Katalysatoren

a) Die spezifischen Oberflächen der Katalysatoren K1 bis K8 wurden mit Hilfe der Stickstoffadorptionsmethode nach DIN ISO 9277 ermittelt.

b) Die kristallographische Phasenzusammensetzungen, z.B. Gehalt an K/Fe-Misch-oxidphasen und Cerdioxid-Kristallitgröße, wurden anhand von Röntgen-Diffraktionsmessungen nach folgender Methode bestimmt:
Die Katalysator-Formkörper wurden mit einer Mühle zu einem feinen Pulver gemahlen. Danach wurden die Proben in einen Standard-Probenhalter (Fa. Bruker AXS GmbH) gefüllt und mit einer Glasplatte glatt gestrichen. Die Proben wurden in einem D8 Advance Diffraktometer (Fa. Bruker AXS GmbH) mit einer variablen Blende (V20 - bestrahlte Probenlänge von 20mm) und einem energiedispersiven Punktdetektor (Sol-X, Fa. Bruker AXS GmbH) im Winkelbereich von 10° bis 55° $2\theta$ (2 Theta) mit einer Schrittweite von 0,02° $2\theta$ (2 Theta) vermessen. Die Auswertung der Daten erfolgte mit der Software TOPAS 4.2 (Fa. Bruker AXS GmbH). Die Phasenzusammensetzung der Katalysatoren umfasste typischerweise variable Anteile von verschiedenen kristallographischen Phasen, z.B. Cerianit ($CeO_2$), Hematit ($Fe_2O_3$), Magnetit ($Fe_3O_4$), $K_2CO_3 \bullet 1,5\,H_2O$, $K_4H_2(CO_3)_3 \bullet 1,5\,H_2O$, $KFe_{11}O_{17}$, $K_2Fe_{10}O_{16}$, $K_6Fe_2O_5$, $K_6Fe_2O_6$, $K_9(FeO_4)_2$ (Raumgruppe C2/c) und $K_{17}Fe_5O_{16}$ (Raumgruppe Cm), $K_2Fe_2O_4$, $KFeO_2$ und gegebenenfalls andere oxidische Phasen, welche abhängig sind von den weiteren beinhalteten Metallverbindungen. Bei allen Phasen wurden die Gitterparameter, Kristallitgröße, und Skalierung verfeinert, sowie bei $KFe_{11}O_{17}$ zusätzlich noch die Gaußsche Komponente der Gitterverzerrung (Strain) und eine March-Dollase Vorzugorientierung in Richtung (001). Der Untergrund wurde mit einem Polynom dritter Ordnung angepasst, und der Probenhöhenfehler wurde verfeinert. Intensitätskorrekturen für die Lorentz-Polarisation wurden berücksichtigt. Die Kristallitgröße ist der von der TOPAS Software mit der Bezeichnung "Lvol FWHM" berechnete Wert.

Tabelle 1: Kalzinierungsbedingungen und Eigenschaften der Katalysatoren K1 bis K8

| Katalysator | Kalzinierungs-temperatur und -dauer | Spezifische Oberfläche | Mittlere $CeO_2$ Kristallitgröße | K/Fe-Mischoxid-phasen | Hämatit |
|---|---|---|---|---|---|
| | | $m^2/g$ | nm | Gew.% | Gew.% |
| K1* | 500°C/ 60 min | 1,6 | 6 | 16 | 69 |
| K2* | 870°C/ 60 min | 1,2 | 38 | 83 | < 1 |
| K3* | 1000°C/ 60 min | 1,2 | 112 | 84 | < 1 |
| K4* | 775°C/ 30 min | 1,6 | 14 | 40 | 44 |
| K5 | 775°C/ 60 min | 1,8 | 16 | 68 | 17 |
| K6 | 800°C/ 30 min | 2,1 | 16 | 61 | 25 |
| K7 | 800°C/ 60 min | 1,8 | 19 | 80 | 5 |

(fortgesetzt)

| Katalysator | Kalzinierungs-temperatur und -dauer | Spezifische Oberfläche | Mittlere CeO$_2$ Kristallitgröße | K/Fe-Mischoxid-phasen | Hämatit |
|---|---|---|---|---|---|
| | | m$^2$/g | nm | Gew.% | Gew.% |
| K8* | 825°C/ 60 min | 1,6 | 24 | 81 | 4 |
| *Vergleichsbeispiele | | | | | |

Beispiele 10 und 11: Katalysator-Herstellung mit kontinuierlicher Kalzinierung

Es wurden Katalysatoren wie in Vergleichsbeispiel 1 beschrieben hergestellt, mit dem Unterschied, dass die Endkalzinierung in einem kontinuierlich betriebenen Drehrohrofen bei unterschiedlichen Temperaturen (870 °C und 890 °C) durchgeführt wurde. Der verwendete Drehrohrofen hatte einen Innendurchmesser von 12 cm und eine Länge von 2 m und wurde elektrisch beheizt. Die Verweilzeit der Katalysatorstränge im Drehrohrofen betrug durchschnittlich 15 min.
Es wurden die Katalysatoren K9 und K10 mit der in Beispiel 1 genannten nominalen Zusammensetzung erhalten. Die Charakterisierung der Katalysatoren erfolgte wie unter Beispiel 9 beschrieben. Die Kalzinierungsbedingungen sowie die physikalischen Eigenschaften der daraus resultierenden Katalysatoren sind in der Tabelle 2 unten zusammengefasst.

Tabelle 2: Kalzinierungsbedingungen und Eigenschaften der Katalysatoren K9 und K10

| Katalysator | Kalzinierung-Temperatur und -Verweilzeit | Spezifische Oberfläche | Mittlere CeO$_2$ Kristallitgröße | K/Fe-Mischoxid-phasen | Hämatit |
|---|---|---|---|---|---|
| | | m$^2$/g | nm | % | % |
| K9 | 870°C/ 15 min | 2,0 | 17 | 60 | 18 |
| K10 | 890°C/ 15 min | 2,1 | 19 | 72 | 7 |

Die Daten aus der Tabellen 1 und 2 zeigen, dass nicht nur Temperatur, sondern auch die Kalzinierungsdauer bzw. Verweilzeit und auch die Art des Kalzinierofens bei der Einstellung des Gehaltes der K/Fe-Mischoxidphasen und der mittlere Cerdioxid-Kristallitgröße eine Rolle spielen.

Beispiel 12: Dehydrierung von Ethylbenzol zu Styrol bei einem Dampf/Ethylbenzol Verhältnis von 1,25 kg/kg

Die Katalysatoren K1 bis K10 aus den Beispielen 1 bis 8 sowie 10 und 11 wurden in der Dehydrierung von Ethylbenzol zu Styrol in Anwesenheit von Wasserdampf eingesetzt. Das erhaltene Katalysatormaterial wurde zerkleinert und gesiebt, wobei eine Fraktion von 0,5 bis 0,7 mm abgetrennt wurde, welche für die weiteren Versuche verwendet wurde.
Pro Katalysator wurden jeweils zwei isothermen Rohrreaktoren mit je 13,3 ml Katalysator der Fraktion mit einer Korngröße von 0,5 bis 0,7 mm gefüllt. Die Reaktoren wurden jeweils bei 620 °C und 1 atm Ausgangsdruck mit 14,6 g/h Ethylbenzol und 18,3 g/h vollentsalztem (VE) Wasser kontinuierlich beaufschlagt, was einem Wasser/Ethylbenzol (D/KW)-Verhältnis von 1,25 kg/kg oder 7,36 Mol/Mol entspricht. Nach Stabilisierung, etwa nach 40 h, wurden aus dem flüssigen Kondensat Proben genommen und gaschromatographisch analysiert. Umsatz, Selektivität und Styrol-Ausbeute wurden für jeden Reaktor bestimmt. Für jeden Katalysator wurde einen Mittelwert über die beiden parallel geführten Reaktoren ermittelt. Die Ergebnisse werden in Tabelle 3 dargestellt.

Tabelle 3: Katalytische Eigenschaften der Katalysatoren K1 bis K10 bei der Umsetzung von Ethylbenzol zu Styrol bei D/KW Verhältnis von 1,25 kg/kg und 620°C.

| Katalysator | Ethylbenzol Umsatz [mol%] | Styrol Selektivität [mol%] | Styrol Ausbeute [mol%] |
|---|---|---|---|
| K1 (Vergleichsbeispiel) | 70,5 | 95,6 | 67,4 |
| K2 (Vergleichs beispiel) | 70,3 | 95,6 | 67,2 |
| K3 (Vergleichs beispiel) | 64,1 | 96,7 | 62,0 |
| K4 (Vergleichs beispiel) | 70,8 | 95,5 | 67,6 |

(fortgesetzt)

| Katalysator | Ethylbenzol Umsatz [mol%] | Styrol Selektivität [mol%] | Styrol Ausbeute [mol%] |
|---|---|---|---|
| K5 | 72,8 | 95,1 | 69,2 |
| K6 | 72,2 | 95,1 | 68,7 |
| K7 | 71,4 | 95,7 | 68,3 |
| K8 (Vergleichs beispiel) | 71,3 | 95,3 | 68,0 |
| K9 | 72,7 | 94,9 | 69,0 |
| K10 | 72,4 | 94,8 | 68,6 |

Ethylbenzol-Umsatz, Styrol-Selektivität und -Ausbeute wurden anhand der folgenden Formeln bestimmt:

$$\text{Umsatz (Mol-\%)} = [A*M_f - B*M_p/(A*M_f)] \times 100$$

$$\text{Selektivität (Mol-\%)} = [D*M_p - C*M_f/(A*M_f - B*M_p)] \times (M_{EB}/M_{ST}) \times 100$$

$$\text{Ausbeute (Mol-\%)} = \text{Umsatz} \times \text{Selektivität} / 100$$

mit:

A: Ethylbenzol-Konzentration am Reaktor-Eingang (Gew.-%)
B: Ethylbenzol-Konzentration am Reaktor-Ausgang (Gew.-%)
C: Styrol-Konzentration am Reaktor-Eingang (Gew.-%)
D: Styrol-Konzentration am Reaktor-Ausgang (Gew.-%)
$M_f$: Mittlere Molmasse der organischen Edukte
$M_p$: Mittlere Molmasse der organischen Produkte
$M_{EB}$: Molmasse Ethylbenzol
$M_{ST}$: Molmasse Styrol

Die oben genannten Angaben bezüglich Konzentration und Molmassen beziehen sich jeweils auf die organische Phase (ohne Wasser).

Die Ergebnisse aus der Tabelle 3 in Kombination mit den Tabellen 1 und 2 zeigen, dass sowohl ein Anteil an K/Fe-Mischoxidphasen von mindestens 20 Gew.-%, insbesondere mindestens 40 Gew.-%, als auch eine Kristallitgröße der Cerdioxid-Partikel im Bereich von 10 bis 30 nm, insbesondere 14 bis 24 nm vorteilhaft sind, um optimale katalytische Leistungen zu erhalten.

Beispielsweise weist der Katalysator K1 (Vergleichsbeispiel 1) eine verhältnismäßig geringe Cerdioxid-Partikelgröße auf, allerdings ist der Gehalt an K/Fe-Mischoxidphasen kleiner als 20 Gew.-%, daher weist Katalysator K1 nur eine vergleichsweise geringe Styrol-Ausbeute auf.

Die Katalysatoren K2 und K3 (Vergleichsbeispiele 2 und 3) weisen dagegen einen hohen Anteil K/Fe-Mischoxidphasen auf, die Cerdioxid-Kristallite der Katalysatoren K2 und K3 sind allerding zu groß (größer als 30 nm), die Aktivität der Katalysatoren K2 und K3 ist daher vergleichsweise niedrig.

Der Katalysator K9 aus Beispiel 9 wurde bei gleicher Temperatur wie der aus dem Vergleichsbeispiel 2 kalziniert, allerdings bei anderen Bedingungen, nämlich 15 min in einem kontinuierlichen Drehrohrofen anstatt 1 h in stationären Muffelofen. Die mittlere Kristallitgröße der Cerdioxid-Partikel des Katalysators K9 ist geringer als die des Katalysators K2, nämlich 17 nm in Vergleich zu 38 nm. Der erfindungsgemäße Katalysator K9 weist eine höhere Aktivität auf im Vergleich zu Katalysator K2.

Der erfindungsgemäße Katalysator K5 (Beispiel 5) (775°C/60 min) wurde im Vergleich zu Katalysator K6 (800°C/30 min) bei einer geringeren Temperatur aber über einen längeren Zeitraum kalziniert. Beide Katalysatoren zeigen eine günstige Aktivität. Beide Katalysatoren weisen eine Ceroxid-Partikelgröße von 16 nm auf, allerdings hat Katalysator K5 einen etwas höheren Gehalt an K/Fe-Mischoxidphasen und folglich eine etwas höhere Aktivität.

**Patentansprüche**

1. Dehydrier-Katalysator enthaltend

    50 bis 90 Gew.-% mindestens einer Eisenverbindung, gerechnet als $Fe_2O_3$;
    1 bis 30 Gew.-% mindestens einer Kaliumverbindung, gerechnet als $K_2O$;
    2 bis 25 Gew.-% mindestens einer Cerverbindung, gerechnet als $CeO_2$;
    0,1 bis 10 Gew.-% mindestens einer Magnesiumverbindung, gerechnet als MgO;
    0,1 bis 10 Gew.-% mindestens einer Calciumverbindung, gerechnet als CaO;
    0,1 bis 10 Gew.-% mindestens einer Molybdänverbindung, gerechnet als $MoO_3$;
    1 bis 1.000 ppm mindestens einer Titanverbindung, gerechnet als $TiO_2$; und
    0 bis 30 Gew.-% mindestens einer weiteren Komponente;
    wobei die mindestens eine Eisenverbindung und die mindestens eine Kaliumverbindung zumindest teilweise in Form von einer oder mehreren K/Fe-Mischoxidphasen der allgemeinen Formel $K_xFe_yO_z$ vorliegen, wobei x eine Zahl von 1 bis 17 ist; y eine Zahl von 1 bis 22 ist, und z eine Zahl von 2 bis 34 ist, wobei der Katalysator mindestens 60 Gew.-%, bezogen auf den gesamten Katalysator, der einen oder mehreren K/Fe-Mischoxidphasen enthält und wobei der Katalysator kristallines Cerdioxid enthält, das eine Kristallitgröße im Bereich von 12 nm bis 22 nm aufweist, wobei der Anteil an K/Fe-Mischoxidphase und die Kristallitgröße mittels Röntgendiffraktometrie bestimmt werden.

2. Dehydrier-Katalysator gemäß Anspruch 1, **dadurch gekennzeichnet, dass** der Katalysator als weitere Komponente 0,0001 bis 10 Gew.-%, mindestens einer Verbindung ausgewählt aus Verbindungen umfassend ein Metall ausgewählt aus der Gruppe bestehend aus Molybdän, Titan, Vanadium und Wolfram, gerechnet als Oxid in der jeweils höchsten Oxidationsstufe, enthält.

3. Verfahren zur Herstellung eines Dehydrier-Katalysators gemäß einem der Ansprüche 1 bis 2, umfassend die folgenden Schritte

    i) Herstellen einer Katalysator-Vormischung durch Vermischen mindestens einer Eisenverbindung, mindestens einer Kaliumverbindung, mindestens einer Cerverbindung und optional weiterer Metallverbindungen, optional weiterer Komponenten und optional mindestens eines Bindemittels mit einem Lösungsmittel;
    ii) Herstellen von Katalysator-Formkörpern aus der in Schritt i) erhaltenen Katalysator-Vormischung;
    iii) Trocknen der Katalysator-Formkörper und Kalzinieren der Katalysator-Formkörper, wobei das Kalzinieren der Katalysator-Formkörper bei Temperaturen von 700 °C bis 800 °C und bei einer Kalzinierungsdauer von 15 min bis 90 min in einem stationären Verfahren erfolgt, oder wobei das Kalzinieren der Katalysator-Formkörper bei Temperaturen von 800 bis 950 °C und bei einer Kalzinierungsdauer von 10 min bis 30 min in einem kontinuierlichen Verfahren erfolgt.

4. Verfahren zur katalytischen Dehydrierung eines Kohlenwasserstoffs, wobei ein Gemisch aus Wasserdampf und mindestens einem Kohlenwasserstoff mit einem Dehydrier-Katalysator gemäß einem der Ansprüche 1 bis 2 in Kontakt gebracht wird.

5. Verfahren zur katalytischen Dehydrierung eines Kohlenwasserstoffs gemäß Anspruch 4, **dadurch gekennzeichnet, dass** ein Gemisch aus Wasserdampf und mindestens einem Kohlenwasserstoff mit einem molaren Dampf/Kohlenwasserstoff-Verhältnis im Bereich von 1 bis 10 eingesetzt wird.

6. Verfahren zur katalytischen Dehydrierung gemäß einem der Ansprüche 4 oder 5, **dadurch gekennzeichnet, dass** es sich bei dem Kohlenwasserstoff um Ethylbenzol handelt.

7. Verwendung eines Dehydrier-Katalysators gemäß einem der Ansprüche 1 bis 2 zur katalytischen Dehydrierung eines Kohlenwasserstoffs.

**Claims**

1. A dehydrogenation catalyst comprising

    from 50 to 90% by weight of at least one iron compound, calculated as $Fe_2O_3$;

from 1 to 30% by weight of at least one potassium compound, calculated as $K_2O$;
from 2 to 25% by weight of at least one cerium compound, calculated as $CeO_2$;
from 0.1 to 10% by weight of at least one magnesium compound, calculated as MgO;
from 0.1 to 10% by weight of at least one calcium compound, calculated as CaO;
from 0.1 to 10% by weight of at least one molybdenum compound, calculated as $MoO_3$;
from 1 to 1000 ppm of at least one titanium compound, calculated as $TiO_2$; and
from 0 to 30% by weight of at least one further component;
wherein the at least one iron compound and the at least one potassium compound are at least partly present in the form of one or more K/Fe mixed oxide phases of the general formula $K_xFe_yO_z$, where x is a number from 1 to 17; y is a number from 1 to 22 and z is a number from 2 to 34, where the catalyst comprises at least 60% by weight, based on the total catalyst, of the one or more K/Fe mixed oxide phases and where the catalyst comprises crystalline cerium dioxide having a crystallite size in the range from 12 nm to 22 nm, where the proportion of K/Fe mixed oxide phase and the crystallite size are determined by means of X-ray diffraction.

2. The dehydrogenation catalyst according to claim 1, wherein the catalyst comprises from 0.0001 to 10% by weight of at least one compound selected from among compounds comprising a metal selected from the group consisting of molybdenum, titanium, vanadium and tungsten, calculated as oxide in the respective highest oxidation state, as further component.

3. A process for producing a dehydrogenation catalyst according to either claim 1 or 2, comprising the following steps

   i) production of a catalyst premix by mixing of at least one iron compound, at least one potassium compound, at least one cerium compound and optionally further metal compounds, optionally further components and optionally at least one binder with a solvent;
   ii) production of shaped catalyst bodies from the catalyst premix obtained in step i);
   iii) drying of the shaped catalyst bodies and calcination of the shaped catalyst bodies, where the calcination of the shaped catalyst bodies is carried out at temperatures of from 700°C to 800°C and for a calcination time of from 15 minutes to 90 minutes in a stationary process, or where the calcination of the shaped catalyst bodies is carried out at temperatures of from 800 to 950°C and for a calcination time of from 10 minutes to 30 minutes in a continuous process.

4. A process for the catalytic dehydrogenation of a hydrocarbon, wherein a mixture of steam and at least one hydrocarbon is brought into contact with a dehydrogenation catalyst according to either claim 1 or 2.

5. The process for the catalytic dehydrogenation of a hydrocarbon according to claim 4, wherein a mixture of steam and at least one hydrocarbon having a molar steam/hydrocarbon ratio in the range from 1 to 10 is used.

6. The process for catalytic dehydrogenation according to either claim 4 or 5, wherein the hydrocarbon is ethylbenzene.

7. The use of a dehydrogenation catalyst according to either claim 1 or 2 for the catalytic dehydrogenation of a hydrocarbon.

**Revendications**

1. Catalyseur de déshydrogénation contenant
   50 à 90 % en poids d'au moins un composé du fer, calculé en tant que $Fe_2O_3$ ;
   1 à 30 % en poids d'au moins un composé du potassium, calculé en tant que $K_2O$ ;
   2 à 25 % en poids d'au moins un composé du cérium, calculé en tant que $CeO_2$ ;
   0,1 à 10 % en poids d'au moins un composé du magnésium, calculé en tant que MgO ;
   0,1 à 10 % en poids d'au moins un composé du calcium, calculé en tant que CaO ;
   0,1 à 10 % en poids d'au moins un composé du molybdène, calculé en tant que $MoO_3$ ;
   1 à 1 000 ppm d'au moins un composé du titane, calculé en tant que $TiO_2$ ; et
   0 à 30 % en poids d'au moins un composant supplémentaire ;
   l'au moins un composé du fer et l'au moins un composé du potassium étant présents au moins partiellement sous forme d'une ou plusieurs phases d'oxyde mixte K/Fe de formule générale $K_xFe_yO_z$, x étant un nombre de 1 à 17 ; y étant un nombre de 1 à 22, et z étant un nombre de 2 à 34, le catalyseur contenant au moins 60 % en poids, par rapport au catalyseur total, de la ou des phases d'oxyde mixte K/Fe et le catalyseur contenant du dioxyde de cérium cristallin qui présente une grosseur de cristallite dans la plage de 12 nm à 22 nm, la proportion de phases d'oxyde mixte K/Fe et la grosseur de cristallite étant déterminées au moyen de diffractométrie des rayons X.

2. Catalyseur de déshydrogénation selon la revendication 1, **caractérisé en ce que** le catalyseur contient en tant que composant supplémentaire 0,0001 à 10 % en poids d'au moins un composé choisi parmi des composés comprenant un métal choisi dans le groupe constitué par le molybdène, le titane, le vanadium et le tungstène, calculé en tant qu'oxyde à chaque fois dans le plus haut état d'oxydation.

3. Procédé pour la préparation d'un catalyseur de déshydrogénation selon l'une quelconque des revendications 1 et 2, comprenant les étapes suivantes

   i) préparation d'un prémélange de catalyseur par mélange d'au moins un composé du fer, d'au moins un composé du potassium, d'au moins un composé du cérium et éventuellement d'autres composés métalliques, éventuellement d'autres composants et éventuellement d'au moins un liant, avec un solvant ;
   ii) préparation de corps moulés de catalyseur à partir du prémélange de catalyseur obtenu dans l'étape i) ;
   iii) séchage des corps moulés de catalyseur et calcination des corps moulés de catalyseur, la calcination des corps moulés de catalyseur étant réalisée à des températures de 700 °C à 800 °C et pendant une durée de calcination de 15 min à 90 min dans un procédé stationnaire, ou la calcination des corps moulés de catalyseur étant réalisée à des températures de 800 à 950 °C et pendant une durée de calcination de 10 min à 30 min dans un procédé en continu.

4. Procédé pour la déshydrogénation catalytique d'un hydrocarbure, dans lequel un mélange de vapeur d'eau et d'au moins un hydrocarbure est mis en contact avec un catalyseur de déshydrogénation selon l'une quelconque des revendications 1 et 2.

5. Procédé pour la déshydrogénation catalytique d'un hydrocarbure selon la revendication 4, **caractérisé en ce qu'**un mélange de vapeur d'eau et d'au moins un hydrocarbure est utilisé avec un rapport molaire vapeur/hydrocarbure dans la plage de 1 à 10.

6. Procédé pour la déshydrogénation catalytique selon l'une quelconque des revendications 4 et 5, **caractérisé en ce que** l'hydrocarbure est l'éthylbenzène.

7. Utilisation d'un catalyseur de déshydrogénation selon l'une quelconque des revendications 1 et 2 pour la déshydrogénation catalytique d'un hydrocarbure.

## FIG.1

## FIG.2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4460706 A
- US 6551958 B
- EP 0894528 A
- EP 1027928 A
- WO 9710898 A
- US 2013165723 A

**In der Beschreibung aufgeführte Nicht-Patentliteratur**

- **HIRANO et al.** *Appl. Catal.,* 1986, vol. 28, 119-130
- **DULAMITA et al.** *Applied Catalysis A: General,* 15. Juni 2005, vol. 287 (1
- **LIAO et al.** *Catalysis Communications,* 15. Mai 2008, vol. 9 (9